# EUROPEAN PATENT SPECIFICATION

(11) **EP 3 586 862 B1**
(45) Date of publication and mention of the grant of the patent: **08.12.2021**
(21) Application number: 19185584.0
(22) Date of filing: 22.10.2013
(51) Int. Cl.: A61K 38/00, A61P 9/10, A61P 9/00

(54) **METHODS FOR REDUCING RISKS ASSOCIATED WITH HEART FAILURE AND FACTORS ASSOCIATED THEREWITH**
VERFAHREN ZUR VERRINGERUNG DER GEFAHREN IM ZUSAMMENHANG MIT HERZINSUFFIZIENZ UND DAMIT ZUSAMMENHÄNGENDEN FAKTOREN
PROCÉDÉS DE RÉDUCTION DES RISQUES ASSOCIÉS À L'INSUFFISANCE CARDIAQUE ET FACTEURS ASSOCIÉS

(30) Priority: 22.10.2012 US 201261716867 P; 13.05.2013 US 201361822752 P; 26.06.2013 US 201361839743 P; 26.06.2013 US 201361839750 P
(43) Date of publication of application: 01.01.2020
(62) Divisional of application: 13849406.7
(73) Proprietor: Stealth Peptides International, Inc., 98000 Monaco (MC); Henry Ford Health Systems, Detroit, MI 48202 (US)
(72) Inventor: WILSON, D. Travis, Newton, MA Massachusetts 02461 (US); SABBAH, Hani N., Detroit, MI Michigan 48202 (US)
(74) Representative: Barker Brettell LLP

(56) References cited:
- EP-A1- 1 890 154
- WO-A1-2011/082324
- WO-A1-2011/082328
- WO-A1-2014/022552
- WO-A1-2014/088631
- WO-A1-2014/210062
- WO-A2-2004/009776
- WO-A2-2011/066371
- WO-A2-2014/209905
- US-A1- 2011 039 766
- US-A1- 2011 082 084
- US-A1- 2013 303 436
- SHEIKH ET AL: "C-reactive Protein as a Predictor of Adverse outcome in Patients with Acute Coronary Syndrome", HEART VIEWS., vol. 13, no. 1, 1 January 2012 (2012-01-01), pages 7-12, XP055261996, DOI: 10.4103/1995-705X.96660
- GUILLERMO TORRE-AMIONE ET AL: "Proinflammatory cytokine levels in patients with depressed left ventricular ejection fraction: A report from the studies of left ventricular dysfunction (SOLVD)", JOURNAL OF THE AMERICAN COLLEGE OF CARDIOLOGY, vol. 27, no. 5, 1 April 1996 (1996-04-01), pages 1201-1206, XP055261998, US ISSN: 0735-1097, DOI: 10.1016/0735-1097(95)00589-7

## Description

### TECHNICAL FIELD

The present technology relates generally to compositions for use in preventing or reducing the risk of heart failure. In particular, the present technology relates to administering the peptide D-Arg-2'6'-Dmt-Lys-Phe-NH₂ in effective amounts to reduce or normalize level of ALCAT 1 in human subjects.

### BACKGROUND

Heart failure is a leading cause of mortality and morbidity worldwide. In the United States, it affects nearly 5 million people and is the only major cardiovascular disorder on the rise. It is estimated that 400,000 to 700,000 new cases of heart failure are diagnosed each year in the U.S. and the number of deaths in the U.S. attributable to this condition has more than doubled since 1979, currently averaging 250,000 annually. Although heart failure affects people of all ages, the risk of heart failure increases with age and is most common among older people. Accordingly, the number of people living with heart failure is expected to increase significantly as the elderly population grows over the next few decades. The causes of heart failure have been linked to various disorders including coronary artery disease, atherosclerosis, past myocardial infarction, hypertension, abnormal heart valves, cardiomyopathy or myocarditis, congenital heart disease, severe lung disease, diabetes, severe anemia, hyperthyroidism, arrhythmia or dysrhythmia.

Heart failure (HF), also called congestive heart failure, is commonly characterized by decreased cardiac output, decreased cardiac contractility, abnormal diastolic compliance, reduced stroke volume, and pulmonary congestion. The clinical manifestations of heart failure reflect a decrease in the myocardial contractile state and a reduction in cardiac output. Apart from deficiencies in cardiac contractility, the HF disease state may arise from left ventricular failure, right ventricular failure, biventricular failure, systolic dysfunction, diastolic dysfunction, and pulmonary effects. A progressive decrease in the contractile function of cardiac muscle, associated with heart disease, often leads to hypoperfusion of critical organs.

Following myocardial infarction there is a dynamic and progressive left ventricle (LV) remodeling that contributes to LV dilation, heart failure, and death. LV remodeling increases LV wall stress, which leads to an increase in oxygen demand. To help compensate for the loss of myocardium and reduced stroke volume, the LV develops global dilation and the non-infarcted wall of the LV develops eccentric hypertrophy. As the ventricle dilates, the dilation process initially helps to compensate for reduced stroke volume. However, eventually progressive dilatation and hypertrophy lead to congestive heart failure. One of the strongest predictors of death one year post myocardial infarction is the volume of the left ventricle.

### SUMMARY

The present invention relates generally to the use of the peptide D-Arg-2'6'-Dmt-Lys-Phe-NH₂ to prevent or reduce the risk of heart failure. The disclosure also relates to reducing the level of C-reactive protein, TNF-alpha, IL-6, or reactive oxygen species ("ROS"), brain natriuretic peptide, and cardiac troponin I in a subject in need thereof by administering a therapeutically effective amount of an aromatic-cationic peptide, or a pharmaceutically acceptable salt thereof, *e.g.,* D-Arg-2',6'-Dmt-Lys-Phe-NH₂. Reducing the level of C-reactive protein, TNF-alpha, IL-6, ROS, or cardiac troponin I may be useful for the treatment or prevention of heart failure, reducing risk factors associated with heart failure, and/or reducing the likelihood (risk) or severity of heart failure in the subject.

The disclosure also relates to the treatment or prevention of left ventricular remodeling in mammals through administration of therapeutically effective amounts of aromatic-cationic peptides to subjects in need thereof. The aromatic-cationic peptides may stabilize mitochondrial biogenesis in cardiac tissues. Administration of aromatic-cationic peptides to a subject in need thereof may lead to a decrease in brain natriuretic peptide (as measure by a decrease in NT-pro BNP), which correlates to a reduction in LV remodeling. Administration of aromatic-cationic peptides to a subject in need thereof may lead to a decrease in cardiac troponin I, which correlates to a reduction in LV remodeling.

The disclosure relates to treating or preventing heart failure comprising administering to the mammalian subject a therapeutically effective amount of an aromatic-cationic peptide or a pharmaceutically acceptable salt thereof, *e.g.,* D-Arg-2',6'-Dmt-Lys-Phe-NH₂. The aromatic-cationic peptide may be a peptide having:
at least one net positive charge;
a minimum of four amino acids;
a maximum of about twenty amino acids;
a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1. The mammalian subject may be a human.

2pₘ is the largest number that is less than or equal to r+1, and a may be equal to pₜ. The aromatic-cationic peptide may be a water-soluble peptide having a minimum of two or a minimum of three positive charges.

In one aspect of the invention, there is provided a composition for use in preventing or reducing the risk of heart failure in a human subject in need thereof, wherein the subject has higher than a control or "normal" level of ALCAT 1, wherein the composition comprises a therapeutically effective amount of the peptide D-Arg-2',6'-Dmt-Lys-Phe-NH₂ or a pharmaceutically acceptable salt thereof.

The subject may have been diagnosed with heart failure. In some embodiments, the heart failure results from hypertension; ischemic heart disease; exposure to a cardiotoxic compound; myocarditis; thyroid disease; viral infection; gingivitis; drug abuse; alcohol abuse; pericarditis; atherosclerosis; vascular disease; hypertrophic cardiomyopathy; acute myocardial infarction; left ventricular systolic dysfunction; coronary bypass surgery; starvation; an eating disorder; or a genetic defect.

In some embodiments, the subject has at least one risk factor associated with heart failure selected from the group consisting of high blood pressure; coronary artery disease; heart attack; irregular heartbeats; diabetes; taking diabetes medications rosiglitazone or pioglitazone; sleep apnea; congenital heart defects; viral infection; alcohol use; obesity; smoking; sedentary lifestyle; high cholesterol; family history of heart failure; stress; and kidney conditions.

In some embodiments, the peptide is administered orally, topically, systemically, intravenously, subcutaneously, intraperitoneally, or intramuscularly.

Additionally or alternatively, in some embodiments, the peptide is administered separately, sequentially or simultaneously administering a cardiovascular agent to the subject. In some embodiments, the cardiovascular agent is one or more of the following agents: an anti-arrhythmia agent, a vasodilator, an anti-anginal agent, a corticosteroid, a cardioglycoside, a diuretic, a sedative, an angiotensin converting enzyme (ACE) inhibitor, an angiotensin II antagonist, a thrombolytic agent, a calcium channel blocker, a throboxane receptor antagonist, a radical scavenger, an anti-platelet drug, a β-adrenaline receptor blocking drug, α-receptor blocking drug, a sympathetic nerve inhibitor, a digitalis formulation, an inotrope, and an antihyperlipidemic drug.

In some embodiments, the peptide is D-Arg-2',6'-Dmt-Lys-Phe-NH₂ in the form of a pharmaceutically acceptable salt. In some embodiments, the salt comprises acetate or trifluoroacetate salt.

The disclosure also relates to a method of improving LV function in a mammalian subject. The subject may have an increased level of one or more of C-reactive protein, reactive oxygen species, interleukin-6, TNF-alpha, and cardiac troponin I. The method comprises: administering to the subject a therapeutically effective amount of the peptide D-Arg-2',6'-Dmt-Lys-Phe-NH₂ or a pharmaceutically acceptable salt thereof.

The mammalian subject may have suffered or is likely to suffer heart failure, myocardial infarction, or other stenotic or vascular event.

The peptide may be D-Arg-2',6'-Dmt-Lys-Phe-NH₂ in the form of a pharmaceutically acceptable salt. The salt may comprise acetate or trifluoroacetate salt.

The disclsoure also relates to methods for reducing the level of Nt-pro BNP and/or cardiac troponin I in a mammalian subject in need thereof, the method comprising: administering to the subject a therapeutically effective amount of the peptide D-Arg-2',6'-Dmt-Lys-Phe-NH₂ or a pharmaceutically acceptable salt thereof. The subject may have suffered acute myocardial infarction. A reduction of Nt-pro BNP and/or cardiac troponin I may be an indicator of an effective prevention, treatment, or amelioration of LV remodeling.

The disclosure also relates to methods for reducing the expression of MLCL AT1 or ALCAT1 in a mammalian subject in need thereof. The method may include administering to the subject a therapeutically effective amount of the peptide D-Arg-2',6'-Dmt-Lys-Phe-NH₂ or a pharmaceutically acceptable salt thereof. The subject may have been diagnosed with heart failure.

The disclosure also relates to methods of increasing the expression of Taz1 in a mammalian subject in need thereof. The method includes administering to the subject a therapeutically effective amount of the peptide D-Arg-2',6'-Dmt-Lys-Phe-NH₂ or a pharmaceutically acceptable salt thereof. The subject may have been diagnosed with heart failure.

The disclosure also relates to methods for reducing the risk of heart failure in a mammalian subject having an increased expression of MLCL AT1 or ALCAT1 and/or decreased expression of Taz1. The method may include administering to the subject a therapeutically effective amount of the peptide D-Arg-2',6'-Dmt-Lys-Phe-NH₂ or a pharmaceutically acceptable salt thereof.

The disclosure also relates to methods for stabilizing cardiolipin remodeling in a mammalian subject having or suspected of having heart failure. The mammalian subject may have an increased expression of MLCL AT1 or ALCAT1 and/or decreased expression of Taz1. The cardiolipin may be 18:2 species of cardiolipin.

The present disclosure also provides methods for reducing the level of cardiac troponin I in a mammalian subject in need thereof. The method may include administering to the subject a therapeutically effective amount of the peptide D-Arg-2',6'-Dmt-Lys-Phe-NH₂ or a pharmaceutically acceptable salt thereof.

The disclosure relates to methods for preventing, treating or ameliorating heart failure in a mammalian subject having an increased level of cardiac troponin I. The method may include administering to the subject a therapeutically effective amount of the peptide D-Arg-2',6'-Dmt-Lys-Phe-NH₂ or a pharmaceutically acceptable salt thereof.

The subject may have been diagnosed with heart failure. The heart failure may result from hypertension; ischemic heart disease; exposure to a cardiotoxic compound; myocarditis; thyroid disease; viral infection; gingivitis; drug abuse; alcohol abuse; pericarditis; atherosclerosis; vascular disease; hypertrophic cardiomyopathy; acute myocardial infarction; left ventricular systolic dysfunction; coronary bypass surgery; starvation; an eating disorder; or a genetic defect.

The subject may have at least one risk factor associated with heart failure selected from the group consisting of high blood pressure; coronary artery disease; heart attack; irregular heartbeats; diabetes; taking diabetes medications rosiglitazone or pioglitazone; sleep apnea; congenital heart defects; viral infection; alcohol use; obesity; smoking; sedentary lifestyle; high cholesterol; family history of heart failure; stress; and kidney conditions.

The peptide may be administered orally, topically, systemically, intravenously, subcutaneously, intraperitoneally, or intramuscularly.

The peptide may be administered separately, sequentially or simultaneously administering a cardiovascular agent to the subject. The cardiovascular agent may be one or more of the following agents: an anti-arrhythmia agent, a vasodilator, an anti-anginal agent, a corticosteroid, a cardioglycoside, a diuretic, a sedative, an angiotensin converting enzyme (ACE) inhibitor, an angiotensin II antagonist, a thrombolytic agent, a calcium channel blocker, a throboxane receptor antagonist, a radical scavenger, an anti-platelet drug, a β-adrenaline receptor blocking drug, α-receptor blocking drug, a sympathetic nerve inhibitor, a digitalis formulation, an inotrope, and an antihyperlipidemic drug.

The peptide may be D-Arg-2',6'-Dmt-Lys-Phe-NH₂ in the form of a pharmaceutically acceptable salt. The salt may comprise acetate or trifluoroacetate salt.

The disclosure also relates to methods for increasing mitochondrial ATP-sensitive potassium channel (mK ATP) activity in a subject in need thereof, the method including administering to the subject a therapeutically effective amount of the peptide D-Arg-2',6'-Dmt-Lys-Phe-NH₂ or a pharmaceutically acceptable salt thereof.

The disclosure also relates to methods for reducing the risk of heart failure in a mammalian subject having a decreased mK ATP activity, the method including administering to the subject a therapeutically effective amount of the peptide D-Arg-2',6'-Dmt-Lys-Phe-NH₂ or a pharmaceutically acceptable salt thereof.

The subject may have been diagnosed with heart failure. The heart failure may result from hypertension; ischemic heart disease; exposure to a cardiotoxic compound; myocarditis; thyroid disease; viral infection; gingivitis; drug abuse; alcohol abuse; pericarditis; atherosclerosis; vascular disease; hypertrophic cardiomyopathy; acute myocardial infarction; left ventricular systolic dysfunction; coronary bypass surgery; starvation; an eating disorder; or a genetic defect. The peptide may be administered orally, topically, systemically, intravenously, subcutaneously, intraperitoneally, or intramuscularly. The pharmaceutically acceptable salt may comprise acetate or trifluoroacetate salt.

The disclosure relates to methods for stabilizing mitochondria in a mammalian subject having or suspected of having heart failure. The mammalian subject may have a decreased activity of mK ATP.

### BRIEF DESCRIPTION OF THE FIGURES

FIG. 1 is a graph showing levels of C-reactive protein as determined by high-sensitivity assay after 6 weeks or 12 weeks of treatment with the aromatic-cationic peptide D-Arg-2',6'-Dmt-Lys-Phe-NH₂ compared to baseline levels and untreated controls.
FIG. 2 is a graph showing levels of ROS after 6 weeks or 12 weeks of treatment with the aromatic-cationic peptide D-Arg-2',6'-Dmt-Lys-Phe-NH₂ compared to baseline levels and untreated controls.
FIG. 3 is a graph showing levels of interleukin-6 (IL-6) after 6 weeks or 12 weeks of treatment with the aromatic-cationic peptide D-Arg-2',6'-Dmt-Lys-Phe-NH₂ compared to baseline levels and untreated controls.
FIG. 4 is a graph showing levels of tumor necrosis factor alpha (TNF-α) after 6 weeks or 12 weeks of treatment with the aromatic-cationic peptide D-Arg-2',6'-Dmt-Lys-Phe-NH₂ compared to baseline levels and untreated controls.
FIG. 5 is a graph showing levels of NT-pro BNP (N-terminal pro-brain natriuretic peptide) after 6 weeks or 12 weeks of treatment with the aromatic-cationic peptide D-Arg-2',6'-Dmt-Lys-Phe-NH₂ compared to baseline levels and untreated controls.
FIG. 6 is a chart showing the effects of D-Arg-2',6'-Dmt-Lys-Phe-NH₂ on cardiolipin content, cardiolipin species 18:2-18:2-18:2-18:2, in a heart failure model.
FIG. 7A is a chart showing the effect of D-Arg-2',6'-Dmt-Lys-Phe-NH₂ on Taz1 expression in a heart failure model.
FIG. 7B is a chart showing the effect of D-Arg-2',6'-Dmt-Lys-Phe-NH₂ on MLCL AT1 expression in a heart failure model.
FIG. 7C is a chart showing the effect of D-Arg-2',6'-Dmt-Lys-Phe-NH₂ on ALCAT1 expression in a heart failure model.

### DETAILED DESCRIPTION

It is to be appreciated that certain aspects, modes, embodiments, variations and features of the invention are described below in various levels of detail in order to provide a substantial understanding of the present invention. The definitions of certain terms as used in this specification are provided below. Unless defined otherwise, all technical and scientific terms used herein generally have the same meaning as commonly understood by one of ordinary skill in the art to which this invention belongs.

As used in this specification and the appended claims, the singular forms "a", "an" and "the" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally-occurring amino acid, *i.e.,* an α-carbon that is bound to a hydrogen, a carboxyl group, an amino group, and an R group, *e.g.,* homoserine, norleucine, methionine sulfoxide, methionine methyl sulfonium. Such analogs have modified R groups *(e.g.,* norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "chronic," with reference to administration, refers to administration of a therapeutic agent, such as an aromatic-cationic peptide, for about 3 days, about 4 days, about 5 days, about 6 days, about 1 week, about 2 weeks, about 3 weeks, 4 weeks, 5 weeks 6 weeks, about 2 months, about 3 months, about 6 months, about 9 months, about 1 year or longer. Chronic administration may include administration once per day, twice per day, 3-5 times per day, every other day, every third day, once per week or once per month.

As used herein, the term "effective amount" or "therapeutically effective amount" refers to a quantity sufficient to achieve a desired therapeutic and/or prophylactic effect, *e.g.,* an amount which results in the decrease of *(e.g.,* normalization of) levels of one or more of, *e.g.,* C-reactive protein, interleukin 6, ROS, TNF-alpha, cardiac troponin I, Nt-pro BNP, MLCL AT1, or ALCAT1 in a subject, and/or an amount which is sufficient to prevent, ameliorate, or treat left ventricle (LV) remodeling and/or improvement of LV function and/or an amount which results in the increase of *(e.g.,* normalization of) expression levels of, *e.g.,* Taz1 and/or increase of mK ATP in a subject in need thereof. In the context of therapeutic or prophylactic applications, the amount of a composition administered to the subject may depend on the levels of C-reactive protein, interleukin 6, ROS, TNF-alpha, Nt-pro BNP, or cardiac troponin I, the expression of MLCL AT 1, ALCAT1, or Taz1, and/or the activity of mK ATP in the subject, the type and severity of the disease and on the characteristics of the individual, such as general health, age, sex, body weight and tolerance to drugs. It may also depend on the degree, severity and type of disease. The skilled artisan will be able to determine appropriate dosages depending on these and other factors. The compositions can also be administered in combination with one or more additional therapeutic compounds. In the methods described herein, the aromatic-cationic peptides may be administered to a subject having one or more signs or symptoms or risk factors of heart failure, such as cardiomegaly, tachypnea, hepatomegaly, and myocardial infarction. For example, a therapeutically effective amount of the aromatic-cationic peptides may include levels in which the level of C-reactive protein, interleukin 6, ROS, TNF-alpha, and/or cardiac troponin I is reduced in a subject after administration. A therapeutically effective amount may prevent, ameliorate, or treat LV remodeling and/or improves LV function. A therapeutically effective amount of the aromatic-cationic peptides may include levels in which the expression of MLCL AT1 or ALCAT1 is reduced in a subject in need thereof after administration. A therapeutically effective amount of an aromatic-cationic peptide may include levels in which the expression of Taz1 is increased in a subject in need thereof after administration. A therapeutically effective amount of the aromatic-cationic peptides may include levels in which the activity of mK ATP is increased. A therapeutically effective amount may also reduce or ameliorate the physiological effects of a heart failure and/or the risk factors of heart failure, and/or the likelihood of heart failure. An effective amount of an aromatic-cationic peptide may be an amount sufficient to decrease levels of brain natriuretic peptide in a subject, *e.g.,* to a normal or control level, for that subject.

As used herein, the terms "congestive heart failure" (CHF), "chronic heart failure," "acute heart failure," and "heart failure" are used interchangeably, and refer to any condition characterized by abnormally low cardiac output in which the heart is unable to pump blood at an adequate rate or in adequate volume. When the heart is unable to adequately pump blood to the rest of the body, or when one or more of the heart valves becomes stenotic or otherwise incompetent, blood can back up into the lungs, causing the lungs to become congested with fluid. If this backward flow occurs over an extended period of time, heart failure can result. Typical symptoms of heart failure include shortness of breath (dyspnea), fatigue, weakness, difficulty breathing when lying flat, and swelling of the legs, ankles or abdomen (edema). Causes of heart failure are related to various disorders including coronary artery disease, systemic hypertension, cardiomyopathy or myocarditis, congenital heart disease, abnormal heart valves or valvular heart disease, severe lung disease, diabetes, severe anemia hyperthyroidism, arrhythmia or dysrhythmia and myocardial infarction. The primary signs of congestive heart failure are cardiomegaly (enlarged heart), tachypnea (rapid breathing; occurs in the case of left side failure) and hepatomegaly (enlarged liver; occurs in the case of right side failure).

As used herein, the term "hypertensive cardiomyopathy" refers to a weakened heart caused by the effects of hypertension (high blood pressure). Over time, uncontrolled hypertension causes weakness of the heart muscle. As hypertensive cardiomyopathy worsens, it can lead to congestive heart failure. Early symptoms of hypertensive cardiomyopathy include cough, weakness, and fatigue. Additional symptoms of hypertensive cardiomyopathy include leg swelling, weight gain, difficulty breathing when lying flat, increasing shortness of breath with activity, and waking in the middle of the night short of breath.

As used herein, the term "left ventricle (LV) remodeling" has the meaning known to those of skill in the art, and refers to a condition, typically following myocardial infarction. Following myocardial infarction there is a dynamic and progressive LV remodeling that contributes to LV dilation, heart failure, and death. Within the first week of a myocardial infarction (MI) the necrotic zone thins and stretches (infarct expansion) contributing to regional dilation of the infarct zone. This phenomenon increases LV wall stress, thus, increasing oxygen demand. To help compensate for the loss of myocardium and reduced stroke volume, the LV develops global dilation and the non-infarcted wall of the LV develops eccentric hypertrophy whereby sarcomeres are added on in a circumferential or lengthwise fashion. As the ventricle dilates this process initially helps to compensate for reduced stroke volume, but eventually progressive dilatation and hypertrophy lead to congestive heart failure. One of the strongest predictors of death one year post MI is the volume of the left ventricle; the more dilated, the greater the chance of death. Metabolic and functional abnormalities of the non-infarcted myocardium and myocardium at the infarct border zone may contribute to the LV remodeling phenomenon. Abnormalities in mitochondrial structure and function can lead to reduced production of ATP in the very muscle needed to support the weakened heart. Therefore, aromatic-cationic peptides, such as D-Arg-2',6'-Dmt-Lys-Phe-NH₂, may be useful to treat, ameliorate and/or prevent LV remodeling, and/or stabilize and/or enhance the function of remaining viable myocardium in a heart failure subject. By way of example, but not by way of limitation, the aromatic-cationic peptide may reduce Nt-pro BNP and/or cardiac troponin I, wherein the reduction of Nt-pro BNP and/or cardiac troponin I correlates to the decrease or reversal of LV remodeling.

As used herein, a "normalized" level of CRP, or TNF-alpha, or IL-6, or ROS, or Nt-pro BNP, or cardiac troponin I levels, and/or MLCL AT1, ALCAT 1, or Taz1 expression, and/or mK ATP activity refers to reducing a subject's CRP level, or TNF-alpha level, or IL-6 level, or ROS level, or Nt-pro BNP levels, or cardiac troponin I, or MLCL AT1, or ALCAT 1 levels and/or increasing Taz1 expression or mK ATP activity to the subject's baseline level or baseline range, or reducing the subject's level to a level or range determined as "normal" or "control" level, *e.g.,* via control studies and/or control sampling of the subject over time, or of an appropriate population *(e.g.,* matched by age, ethnicity, disease state, drug treatment regime, weight, sex, etc.). As used herein "control level" refers to a level considered average or normal for the subject, or for an appropriate population of subjects.

As used herein "reducing" a subject's CRP level, or TNF-alpha level, or IL-6 level, or ROS level, or Nt-pro BNP level, or cardiac troponin I level, or MLCL AT1 expression level, or ALCAT 1 expression level means lowering the level of CRP, or TNF-alpha, or IL-6, or ROS, or Nt-pro BNP, or cardiac troponin I, or MLCL AT1, or ALCAT1 expression level in the subject *(e.g.,* a subject's blood CRP level). Reducing CRP level or TNF-alpha level or IL-6 level or ROS level, or Nt-pro BNP level, or cardiac troponin I level, or MLCL AT1, or ALCAT1 level may be a reduction by about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or more.

As used herein "increasing" a subject's Taz1 expression level or mK ATP activity means increasing the level of Taz1 *(e.g.,* a subject's Taz1 expression level in left ventricular myocardium) or increasing the activity of mK ATP in the subject. Increasing Taz1 expression level and/or mK ATP activity may be an increase by about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, or about 110% or more, *e.g.,* from a baseline or control level. Alternatively, or additionally, increasing Taz1 expression level may be measured as attenuating the reduction of Taz1 by about 0.25 fold to about 0.5 fold, or about 0.5 fold to about 0.75 fold, or about 0.75 fold to about 1.0 fold, or about 1.0 fold to about 1.5 fold, *e.g.,* as compared to a baseline or control level.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the terms "polypeptide," "peptide," and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, the term "simultaneous" therapeutic use refers to the administration of at least two active ingredients by the same route and at the same time or at substantially the same time.

As used herein, the term "separate" therapeutic use refers to an administration of at least two active ingredients at the same time or at substantially the same time by different routes.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

As used herein, the terms "treating" or "treatment" or "alleviation" refers to therapeutic treatment, wherein the object is to prevent or slow down (lessen) the targeted pathologic condition or disorder. A subject is successfully "treated" for heart failure if, after receiving a therapeutic amount of the aromatic-cationic peptides according to the methods described herein, the subject shows observable and/or measurable reduction in or absence of one or more signs and symptoms of heart failure, such as, *e.g.,* cardiac output, myocardial contractile force, cardiomegaly, tachonea, and/or hepahemogaly. Treating heart failure, as used herein, also refers to treating any one or more of the conditions underlying heart failure, including, without limitation, decreased cardiac contractility, abnormal diastolic compliance, reduced stroke volume, pulmonary congestion, and decreased cardiac output. The terms also apply to a reduction in C-reactive protein, interleukin 6, ROS, TNF-alpha, cardiac troponin I levels, Nt-pro BNP, MLCL AT1 and/or ALCAT 1 in those subjects having higher than a control or "normal" level of C-reactive protein, interleukin 6, ROS, TNF-alpha, cardiac troponin I levels, Nt-pro BNP, MLCL AT1 and/or ALCAT 1. The terms also apply to an increase in Taz1 expression and/or increased mK ATP activity in those subjects having lower than a control or "normal" level of Taz1 or lower activity of mK ATP. Additionally, or alternatively, the terms apply to an observable and/or measurable reduction in or absence of one or more signs and symptoms associated with LV remodeling, such as, *e.g.,* LV stroke volume, improved LV ejection fraction, improved fractional shortening, reduced infarct expansion, improved hemodynamics, reduced scar formation in LV myocardium, and reduced lung volumes. It is also to be appreciated that the various modes of treatment or prevention of medical conditions as described are intended to mean "substantial," which includes total but also less than total treatment or prevention, and wherein some biologically or medically relevant result is achieved.

As used herein, "prevention" or "preventing" of a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample. As used herein, preventing heart failure includes preventing the initiation of heart failure, delaying the initiation of heart failure, preventing the progression or advancement of heart failure, slowing the progression or advancement of heart failure, delaying the progression or advancement of heart failure, and reversing the progression of heart failure from an advanced to a less advanced stage. As used herein, prevention of heart failure also includes preventing a recurrence of heart failure. As used herein, preventing LV remodeling includes preventing the initiation of LV remodeling, delaying the initiation of LV remodeling, preventing the progression or advancement of LV remodeling, slowing the progression or advancement of LV remodeling, delaying the progression or advancement of LV remodeling, and reversing the progression of LV remodeling from an advanced to a less advanced stage.

As used herein, "net charge" refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

As used herein, the term "pharmaceutically acceptable salt" referees a salt prepared from a base or an acid which is acceptable for administration to a patient, such as a mammal *(e.g.,* salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein.

### C-Reactive Protein

As used herein "C-reactive protein" (CRP) refers to a pentameric polypeptide composed of five identical subunits, which is a member of the pentraxin family of proteins. The CRP subunit is expressed as a 224-amino acid pro-polypeptide; an 18 amino-acid leader sequence is removed to form a mature 206-amino acid CRP unit. Exemplary, non-limiting sequences of the 224-amino acid CRP precursor are provided below in Table 1 (Accession Numbers NP_000558 and CAA39671, respectively).

| **Table 1: Exemplary CRP precursor amino acid sequences** |
|---|
| SEQ ID NO: 1 |
| mekllcflvl tslshafgqt dmsrkafvfp kesdtsyvsl kapltkplka ftvclhfyte lsstrgysif syatkrqdne ilifwskdig ysftvggsei lfevpevtva pvhictswes asgivefwvd gkprvrkslk kgytvgaeas iilgqeqdsf ggnfegsqsl vgdignvnmw dfvlspdein tiylggpfsp nvlnwralky evqgevftkp qlwp |
| SEQ ID NO: 2 |
| mekllcflvl tslshafgqt dmsrkafvfp kesdtsyvsl kapltkplka ftvclhfyte lsstrgtvfs rmpprdktmr ffifwskdig ysftvggsei lfevpevtva pvhictswes asgivefwvd gkprvrkslk kgytvgaeas iilgqeqdsf ggnfegsqsl vgdignvnmw dfvlspdein tiylggpfsp nvlnwralky evqgevftkp qlwp |

CRP is an acute phase reactant, which is produced by the liver in response to inflammatory stimuli and which circulates in the blood. CRP levels rise in response to acute or chronic inflammation, such as but not limited to inflammation due to infection *(e.g.,* bacterial, viral or fungal), rheumatic and other inflammatory diseases, malignancy, tissue injury or necrosis. Plasma CRP levels of can increase *(e.g.,* 100-fold or more) after severe trauma, bacterial infection, inflammation, surgery or neoplastic proliferation. CRP levels rapidly increase within hours after tissue injury, and it is suggested that CRP is part of the innate immune system and contributes to host defense. Regarding CRP function and the immune system, CRP has been shown to increase LDL uptake into macrophages and enhance the ability of macrophages to form foam cells; inhibit endothelial nitric oxide synthase expression in endothelial cells; increase plasminogen activator inhibitor-1 expression and activity; activate macrophages to secrete tissue factor; up regulate the expression of adhesion molecules in endothelial cells to attract monocytes to the site of injury. Thus, CRP levels have been utilized as a marker for inflammation and immune response.

In addition, CRP levels have been correlated with vascular sclerosis and cardiovascular disease or cardiovascular events, such as heart failure and myocardial infarction. Since cardiovascular disease is at least in part an inflammatory process, CRP has been investigated, for example, in the context of arteriosclerosis and subsequent vascular disorders. For example, a chronic, low-level increase of CRP was found to be predictive of the risk of future cardiovascular events, including myocardial infarction, ischemic stroke, peripheral vascular disease and sudden cardiac death. Based on multiple epidemiological and intervention studies, minor CRP elevation (as determined by high-sensitivity CRP assays, "hsCRP") has been shown to be associated with future cardiovascular risk. HsCRP is discussed in more detail below.

In addition to its role as a cardiovascular risk marker, CRP has also been shown to participate directly in atherogenesis, and high levels of CRP mRNA have been found in atherosclerotic plaques. It has been show that CRP is produced by human artery smooth muscle cells of atherosclerotic lesions in response to inflammatory cytokines; thus, locally produced CRP may participate directly in aspects of atherogenesis, promoting the development of cardiovascular complications.

Treatment with an aromatic-cationic peptide, such as, e.g., D-Arg-2'6'-Dmt-Lys-Phe-NH₂, may decrease C-reactive protein levels in the myocardium in mammalian subjects that have suffered or are at risk of suffering heart failure.

C-reactive protein levels may be decreased by about 0.25 fold to about 0.5 fold, or about 0.5 fold to about 0.75 fold, or about 0.75 fold to about 1.0 fold, or about 1.0 fold to about 1.5 fold, or about 1.5 fold to about 2.0 fold, or about 2.0 fold to about 3.0 fold, or about 3.0 fold to about 5.0 fold, or about 5.0 fold to about 6.0 fold.

### Determination of CRP Levels

There are three broad categories of CRP assays as recognized by the U.S. Food and Drug Administration (FDA): (1) Conventional C-Reactive Protein (CRP) assays; (2) High sensitivity C-Reactive Protein (hsCRP) assays; and (3) Cardiac C-Reactive Protein (cCRP) assays.

### Conventional CRP assays

Conventional CRP assays typically include qualitative, semi-quantitative and quantitative assays, with indications for use for evaluation of infection, tissue injury, and inflammatory disorders. These assays provide information for the diagnosis, therapy, and monitoring of inflammatory diseases. As discussed previously, CRP is one of the cytokine-induced "acute-phase" proteins whose blood levels rise during a general, unspecific response to infections and non-infectious inflammatory processes. For conventional CRP assays, test values are typically considered clinically significant at levels above 10 mg/L. In apparently healthy person's blood CRP levels are below 5 mg/L, while in various conditions this threshold is often exceeded within four to eight hours after an acute inflammatory event, with CRP values reaching approximately 20 to 500 mg/L. CRP is a more sensitive and more reliable indicator of acute inflammatory processes than the erythrocyte sedimentation rate (ESR) and leukocyte count. Blood CRP levels rise more rapidly than ESR, and after the disease has subsided CRP values rapidly fall and reach the reference interval often days before ESR has returned to normal.

### High sensitivity CRP assays

High sensitivity CRP assays have a range of measurement that extends below the measurement range typical of most conventional CRP assays. This lower range of measurement expands the indications for use to include, by way of example but not by way of limitation, the evaluation of conditions thought to be associated with inflammation in otherwise seemingly healthy individuals, and to evaluate cardiac risk in subjects suffering from or at risk of heart failure. Typically, hsCRP assays measure CRP levels from less than 1 mg/L *(e.g.,* as low as 0.04 mg/ml) to greater than or equal to 10 mg/L. As used herein and as is common in the art "high sensitivity CRP" or "hsCRP" refers to the assay, and also refers to the CRP levels as determined by hsCRP assay. Thus, the statement "a subject's hsCRP level is less than 1 mg/L" means that the subjects CRP level, as determined by a high sensitivity CRP assay, is less than 1 mg/L. Thus, the subject's CRP level is less than 1 mg/L.

### Cardiac C-Reactive Protein (cCRP) assays

Pursuant to FDA guidelines, cardiac CRP assays are indicated for use as an aid in the identification and stratification of individuals at risk for future cardiovascular disease. When used in conjunction with traditional clinical laboratory evaluation of acute coronary syndromes, cCRP may be useful as an independent marker of prognosis for recurrent events in patients with stable coronary disease or acute coronary syndrome. Cardiac CRP assays, like hsCRP assays, have measurement ranges that extend below the measurement range typical of most conventional CRP assays. The difference between hsCRP and cCRP is not the analyte itself or even the method of the assay, but the additional performance validation required by the FDA to support the expanded intended use in the evaluation of coronary disease. Accordingly, cCRP assays are a species of hsCRP assay. While hsCRP assays are useful to correlate cardiac risk and are used in the art to do so, they are simply not recognized by the FDA for such uses.

### Sample Source C-Reactive Protein

While C-reactive protein levels are typically tested via a subject's blood sample *(e.g.,* whole blood, plasma, serum), the present disclosure is not intended to be limited by sample type. For example, C-reactive protein levels may be determined by evaluating any fluid or tissue sample of a subject, know to or suspected of containing C-reactive protein. Non-limiting examples include plasma, serum, whole blood, urine, sputum, semen, cerebrospinal fluid, pericardial fluid, peritoneal fluid, pleural fluid, synovial fluid, stool samples and nasal aspirates.

### Interleukin-6

Interleukin-6 (IL-6) belongs to a family of pleiotropic and evolutionary conserved cytokines involved in the regulation of stem cells, hematopoiesis, thrombopoiesis, macrophage function, neuron function, acute phase response, bone metabolism, and cardiac hypertrophy. The family includes, besides IL-6, the cytokines IL-11, cardiotrophin-1, oncostatin-M, leukemia-inhibitory factor, and ciliary neurotrophic factor, which all utilize a common signal-transducing component named gp130 besides their cytokine specific receptors. Recently, elevated soluble IL-6 receptor (IL-6R) levels in heart failure have been reported. Circulating IL-6 exerts a negative inotropic influence on isolated papillary muscle preparations in the hamster model and in atrial strips in humans, potentially modulated by the nitric oxide, β-adrenoceptor pathway, and the ceramide-sphingomyelin pathway. Elevated serum IL-6 levels correlate with reduced contractility, elevated preload, elevated heart rate, and reduced afterload in patients with impaired left ventricular function.

Regarding the source of IL-6 production, recent murine transgene and knockout data suggest that an intracardiac IL-6/gp130 system exists and is an essential component in the compensatory response to hemodynamic overload. Human myocardium has been suggested to be a source of IL-6 during myocardial infarction, ischemia, reperfusion, rejection, and heart failure. *See* Wollert, K. and Drexler, H., "The role of interleukin-6 in the failing heart", Heart Fail Rev., 6(2): 95-103 (2001).

Treatment with an aromatic-cationic peptide, such as, e.g., D-Arg-2'6'-Dmt-Lys-Phe-NH₂, may decrease IL-6 levels in the myocardium in mammalian subjects that have suffered or are at risk of suffering heart failure.

IL-6 levels may bee decreased by about 0.25 fold to about 0.5 fold, or about 0.5 fold to about 0.75 fold, or about 0.75 fold to about 1.0 fold, or about 1.0 fold to about 1.5 fold, or about 1.5 fold to about 2.0 fold, or about 2.0 fold to about 3.0 fold, or about 3.0 fold to about 5.0 fold, or about 5.0 fold to about 8.0 fold.

### Tumor necrosis factor alpha

Tumor necrosis factor alpha (TNF-alpha or TNF-α) is a cytokine involved in systemic inflammation and is a member of a group of cytokines that stimulate the acute phase reaction. It is produced chiefly by activated macrophages, although it can be produced by many other cell types as CD4+ lymphocytes, NK cells and neurons.

In patients with advanced congestive heart failure (CHF), elevated levels of circulating TNF-alpha and soluble TNF receptors have been found. The pathophysiological implications of activation of the TNF system in CHF seem to rely mainly on its effects on the heart and the endothelium. TNF-alpha exerts a negative inotropic effect both directly and indirectly, this latter being mediated by enhancement of nitric oxide production. Moreover, TNF-alpha has been suggested to trigger the apoptotic process in cardiac myocytes. There is consensus on the detrimental role played by TNF-alpha in CHF further supported by the evidence of a temporal association between TNF activation and transition from asymptomatic to symptomatic CHF. *See* Ceconi et al., "Tumor necrosis factor in congestive heart failure: a mechanism of disease for the new millennium?" Proq. Cardiovasc. Dis., 41(Suppl 1): 25-30 (1998).

Treatment with an aromatic-cationic peptide, such as, e.g., D-Arg-2'6'-Dmt-Lys-Phe-NH₂, decreases TNF-alpha levels in the myocardium in mammalian subjects that have suffered or are at risk of suffering heart failure.

TNF-alpha levels may be decreased by about 0.25 fold to about 0.5 fold, or about 0.5 fold to about 0.75 fold, or about 0.75 fold to about 1.0 fold, or about 1.0 fold to about 1.5 fold, or about 1.5 fold to about 2.0 fold, or about 2.0 fold to about 3.0 fold, or about 3.0 fold to about 5.0 fold, or about 5.0 fold to about 8.0 fold.

### Reactive oxygen species

Reactive oxygen species (ROS) are chemically reactive molecules containing oxygen. Examples include oxygen ions and peroxides. ROS form as a natural byproduct of the normal metabolism of oxygen and have important roles in cell signaling and homeostasis. However, during times of environmental stress ROS levels can increase dramatically. The increased of ROS may result in significant damage to cell structures, cumulatively known as oxidative stress.

There is evidence suggesting that oxygen-derived free radicals are involved in the pathogenesis of CHF. Studies suggest that the highly toxic radical species damage subcellular membranes leading to the disruption in excitation-contractile coupling and eventually the dysfunction of the myocardium. In addition, these radicals destroy nitric oxide, a potent signaling molecule responsible for maintaining cardiovascular tone. Antioxidants hold great promise in minimizing the damage occurring as a result of the excessive generation of the free radicals during ischemia/reperfusion injury and CHF.

Treatment with an aromatic-cationic peptide, such as, e.g., D-Arg-2'6'-Dmt-Lys-Phe-NH₂, may decrease ROS levels in the myocardium in mammalian subjects that have suffered or are at risk of suffering heart failure.

ROS may be decreased by about 0.25 fold to about 0.5 fold, or about 0.5 fold to about 0.75 fold, or about 0.75 fold to about 1.0 fold, or about 1.0 fold to about 1.5 fold, or about 1.5 fold to about 2.0 fold, or about 2.0 fold to about 3.0 fold, or about 3.0 fold to about 5.0 fold, or about 5.0 fold to about 8.0 fold.

### Brain natriuretic peptide

Brain natriuretic peptide (BNP) is a 32-amino acid polypeptide secreted by the ventricles of the heart in response to excessive stretching of cardiomyocytes. The release of BNP is modulated by calcium ions. BNP in humans is produced mainly in the cardiac ventricles.

BNP is synthesized as a 134-amino acid preprohormone (preproBNP), encoded by the human gene NPPB. The removal of the 25-residue N-terminal signal peptide generates the prohormone, proBNP, which is stored intracellularly as an O-linked glycoprotein. ProBNP is subsequently cleaved by a specific convertase, *i.e.,* furin or corin, into NT-pro BNP, which is a 76-amino acid biologically inactive polypeptide, and BNP, the biologically active 32-amino acid polypeptide. ProBNP and NT-pro BNP are secreted into the blood in equimolar amounts.

BNP binds to and activates the atrial natriuretic factor in a fashion similar to atrial natriuretic peptide (ANP) but with 10-fold lower affinity. The biological half-life of BNP, however, is twice as long as that of ANP, and that of NT-proBNP is even longer, making these peptides better targets than ANP for diagnostic blood testing.

The physiologic actions of BNP are similar to those of ANP and include decrease in systemic vascular resistance and central venous pressure as well as an increase in natriuresis. The net effect of BNP and ANP is a decrease in blood volume, which lowers systemic blood pressure and afterload, yielding an increase in cardiac output, partly due to a higher ejection fraction.

BNP or NT-pro BNP can be used for screening and prognosis of heart failure. Elevated BNP or NT-pro BNP levels can indicate risk for heart failure or indicate occurrence of acute heart failure. Additionally, both are typically increased in patients with left ventricular dysfunction reduction of BNP concentration after treatments, *e.g.,* with ACE inhibitors or β blockers, may reflect the reversal or prevention of the LV remodeling process.

Treatment with an aromatic-cationic peptide, such as, *e.g.,* D-Arg-2'6'-Dmt-Lys-Phe-NH₂, may decrease NT-pro BNP in the myocardium in mammalian subjects that have suffered or are at risk of suffering heart failure.

NT-pro BNP may decrease by about 0.25 fold to about 0.5 fold, or about 0.5 fold to about 0.75 fold, or about 0.75 fold to about 1.0 fold, or about 1.0 fold to about 1.5 fold, or about 1.5 fold to about 2.0 fold.

### Cardiac Troponin I

Troponin is a complex of three regulatory proteins (troponin C, troponin I, and troponin T) that is integral to muscle contraction in skeletal and cardiac muscle, but not smooth muscle. Troponin T and troponin I isoforms from cardiac muscle are structurally different from the corresponding isoforms found in skeletal muscle.

Troponin T binds to tropomyosin, interlocking them to form a troponin-tropomyosin complex. Troponin I binds to actin in thin myofilaments to hold the troponin-tropomyosin complex in place and decreases troponin C affinity for calcium, thus inhibiting troponin-tropomyosin interactions. Troponin C binds to calcium ions and plays the main role in Ca²⁺ dependent regulation of muscle contraction.

Since cardiac troponin (cTn) is structurally different from skeletal troponin, the cTn subunits can be measured as a specific biomarker for myocardial injury. cTn is released as a result of myocyte necrosis, myocyte apoptosis, or cardiac troponin degradation, all of which would indicate the worsening of cardiac dysfunction and/or progression of heart failure. Multiple studies have evaluated the association between elevated circulating cTn and adverse clinical outcomes in various HF populations. Despite variations in study design, patient populations, and assay characteristics, there has been a consistent association between cTn elevation and worsened outcomes. Kociol et al., Journal of the American College of Cardiology, 56(14): 1071-78 (Sept. 2010).

Additionally, studies showed that elevated levels of cardiac troponin I may contribute to the progression of heart failure. *See, e.g.,* Göser et al., Circulation, 114: 1693-1702 (2006). Elevated levels of cardiac troponin I lead to the formation of cardiac troponin autoantibodies. Increased cardiac troponin autoantibodies increased cardiac inflammation and increased expression of inflammatory cytokines. Increased cardiac troponin autoantibodies produced cardiomyopathic phenotype characterized by myocardial fibrosis, left ventricular dilation, and impaired cardiac function.

Treatment with an aromatic-cationic peptide, such as, e.g., D-Arg-2'6'-Dmt-Lys-Phe-NH₂, may decrease cardiac troponin I in the myocardium in mammalian subjects that have suffered or are at risk of suffering heart failure.

Cardiac troponin I may be decreased by about 0.25 fold to about 0.5 fold, or about 0.5 fold to about 0.75 fold, or about 0.75 fold to about 1.0 fold, or about 1.0 fold to about 1.5 fold, or about 1.5 fold to about 2.0 fold.

### Mitochondrial ATP-sensitive potassium channel (mK ATP)

The reduced form of nicotinamide adenine dinucleotide phosphate (NADPH) is increased in the failing heart and leads to reduced activation of the mitochondria ATP-sensitive potassium channels (mK ATP). The reduced mK ATP activity leads to ionic deregulation in the mitochondrial environment with subsequent matrix contraction and reduced ATP production. Increased activity or "opening" of mK ATP improves oxidative phosphorylation by promoting matrix swelling, maintains the structure of the inner mitochondrial membrane, preserves the low permeability of the outer membrane to ADP, and permits "efficient" energy transfers between mitochondrial and myofibrillar ATPase.

Treatment with an aromatic-cationic peptide, such as, *e.g.,* D-Arg-2'6'-Dmt-Lys-Phe-NH₂, may increase the mK ATP activity in the myocardium in mammalian subjects that have suffered or are at risk of suffering heart failure.

mK ATP activity may be increased by about 0.25 fold to about 0.5 fold, or about 0.5 fold to about 0.75 fold, or about 0.75 fold to about 1.0 fold, or about 1.0 fold to about 1.5 fold, or about 1.5 fold to about 2.0 fold.

### Cardiolipin

Cardiolipin (cardiolipin) is an important component of the inner mitochondrial membrane, where it constitutes about 20% of the total lipid composition. In mammalian cells, cardiolipin is found almost exclusively in the inner mitochondrial membrane where it is essential for the optimal function of enzymes involved in mitochondrial metabolism.

Cardiolipin is a species of diphosphatidylglycerol lipid comprising two phosphatidylglycerols connected with a glycerol backbone to form a dimeric structure. It has four alkyl groups and potentially carries two negative charges. As there are four distinct alkyl chains in cardiolipin, the molecule has the potential for great complexity. However, in most animal tissues, cardiolipin contains 18-carbon fatty alkyl chains with 2 unsaturated bonds on each of them. It has been proposed that the (18:2) in the four acyl chain configuration is an important structural requirement for the high affinity of cardiolipin to inner membrane proteins in mammalian mitochondria. However, studies with isolated enzyme preparations indicate that its importance may vary depending on the protein examined.

Each of the two phosphates in the molecule can capture one proton. Although it has a symmetric structure, ionization of one phosphate happens at different levels of acidity than ionizing both, with pK1 =3 and pK2 > 7.5. Hence, under normal physiological conditions (a pH of approximately 7.0), the molecule may carry only one negative charge. Hydroxyl groups (-OH and -O-) on the phosphate form stable intramolecular hydrogen bonds, forming a bicyclic resonance structure. This structure traps one proton, which is conducive to oxidative phosphorylation.

During the oxidative phosphorylation process catalyzed by Complex IV, large quantities of protons are transferred from one side of the membrane to another side causing a large pH change. Without wishing to be bound by theory, it has been suggested that cardiolipin functions as a proton trap within the mitochondrial membranes, strictly localizing the proton pool and minimizing pH in the mitochondrial intermembrane space. This function is thought to be due to the unique structure of cardiolipin, which, as described above, can trap a proton within the bicyclic structure while carrying a negative charge. Thus, cardiolipin can serve as an electron buffer pool to release or absorb protons to maintain the pH near the mitochondrial membranes.

In addition, cardiolipin has been shown to play a role in apoptosis. An early event in the apoptosis cascade involves cardiolipin. As discussed in more detail below, a cardiolipin-specific oxygenase produces cardiolipin-hydroperoxides which causes the lipid to undergo a conformational change. The oxidized cardiolipin then translocates from the inner mitochondrial membrane to the outer mitochondrial membrane where it is thought to form a pore through which cytochrome c is released into the cytosol. Cytochrome c can bind to the IP3 receptor stimulating calcium release, which further promotes the release of cytochrome c. When the cytoplasmic calcium concentration reaches a toxic level, the cell dies. In addition, extra-mitochondrial cytochrome c interacts with apoptotic activating factors, causing the formation of apoptosomal complexes and activation of the proteolytic caspase cascade.

Other roles proposed for cardiolipin are: 1) participation in stabilization of the physical properties of the membrane (Schlame *et al.,* 2000; Koshkin and Greenberg, 2002; Ma *et al.,* 2004), for example, membrane fluidity and osmotic stability and 2) participation in protein function via direct interaction with membrane proteins (Schlame *et al.,* 2000; Palsdottir and Hunte, 2004). Cardiolipin has been found in tight association with inner membrane protein complexes such as the cytochrome *bc1* complex (complex III). As well, it has been localized to the contact sites of dimeric cytochrome c oxidase, and cardiolipin binding sites have also been found in the ADP/ATP carrier (AAC; for review see Palsdottir and Hunte, 2004). Recent work also suggests a role of cardiolipin in formation of respiratory chain super complexes (respirasomes).

The major tetra-acyl molecular species are 18:2 in each of the four fatty acyl positions of the cardiolipin molecule (referred to as the 18:2-18:2-18:2-18:2 cardiolipin species). Remodeling of cardiolipin is essential to obtain this enrichment of cardiolipin with linoleate because cardiolipin synthase has no molecular species substrate specificity for cytidine-5'-diphosphate-1,2-diacyl-sn-glycerol. In addition, the species pattern of cardiolipin precursors is similar enough to imply that the enzymes of the cardiolipin synthetic pathway are not molecular species-selective. Alterations in the molecular composition of cardiolipin are associated with various disease states.

Remodeling of cardiolipin occurs via at least three enzymes. Mitochondrial cardiolipin is remodeled by a deacylation-reacylation cycle in which newly synthesized cardiolipin is rapidly deacylated to monolysocardiolipin (MLCL) and then reacylated back to cardiolipin. MLCL AT1 is responsible for the deacylation and ALCAT1 is responsible for the reacylation. In addition to these mitochondrial and microsomal acyltransferase activities, mitochondrial cardiolipin may be remodeled by a mitochondrial cardiolipin transacylase. Tafazzin (TAZ1) is a cardiolipin transacylase that specifically remodels mitochondrial cardiolipin with linoleic acid.

### Taz1

Tafazzin (Taz1) is a protein that in humans is encoded by the *TAZ* gene. Taz1 functions as a phospholipid-lysophospholipid transacylase. Taz1 is highly expressed in cardiac and skeletal muscle and is involved in the metabolism of cardiolipin.

Taz1 is involved in the maintenance of the inner membrane of mitochondria. These proteins are involved in maintaining levels of cardiolipin, which is essential for energy production in the mitochondria.

Some mutations in the *TAZ* gene cause a condition called X-linked dilated cardiomyopathy. This is a condition in which the heart becomes so weakened and enlarged that it cannot pump blood efficiently, leading to heart failure. The decreased heart function can negatively affect many body systems and lead to swelling in the legs and abdomen, fluid in the lungs, and an increased risk of blood clots.

Another mutation in the *TAZ* gene causes a condition called isolated non-compaction of left ventricular myocardium (INVM). This condition occurs when the lower left chamber of the heart (left ventricle) does not develop correctly. The heart muscle is weakened and cannot pump blood efficiently, often leading to heart failure. Sometimes abnormal heart rhythms (arrhythmias) can also occur.

Treatment with an aromatic-cationic peptide, such as, *e.g.,* D-Arg-2'6'-Dmt-Lys-Phe-NH₂, may increase the expression of Taz1 in the myocardium in mammalian subjects that have suffered or are at risk of suffering heart failure.

Taz1 expression level may be increased by about 0.25 fold to about 0.5 fold, or about 0.5 fold to about 0.75 fold, or about 0.75 fold to about 1.0 fold, or about 1.0 fold to about 1.5 fold.

### MLCL AT1

Monolysocardiolipin acyltransferase (MLCL AT1) catalyzes the acylation of MLCL to cardiolipin in mammalian tissues.

Treatment with an aromatic-cationic peptide, such as, e.g., D-Arg-2'6'-Dmt-Lys-Phe-NH₂, may decrease the expression of MLCL AT1 in the myocardium in mammalian subjects that have suffered or are at risk of suffering heart failure.

Reducing MLCL AT1 expression level may be a reduction measured by about 1 fold to about 1.5 fold reduction, or about 1.5 fold to about 2.0 fold reduction, or about 2.0 fold to about 2.5 fold reduction, or about 2.5 fold to about 3.0 fold reduction.

### ALCAT1

Acyl-CoA lysocardiolipin acyltransferase 1 (ALCAT1) was initially identified as a microsomal lysocardiolipin acyltransferase. ALCAT1 possesses acyltransferase activities toward lysophosphatidylinositol (LPI) and lysophosphatidylglycerol (LPG).

ALCAT1 recognizes both monolysocardiolipin and dilysocardiolipin as substrates with a preference for linoleoyl-CoA and oleoyl-CoA as acyl donors. ALCAT1 acts as a remodeling enzyme for cardiolipin.

Treatment with an aromatic-cationic peptide, such as, e.g., D-Arg-2'6'-Dmt-Lys-Phe-NH₂, may decrease the expression of ALCAT1 in the myocardium in mammalian subjects that have suffered or are at risk of suffering heart failure.

Reducing ALCAT1 expression level may be a reduction measured by about 1 fold to about 1.5 fold reduction, or about 1.5 fold to about 2.0 fold reduction, or about 2.0 fold to about 2.5 fold reduction, or about 2.5 fold to about 3.0 fold reduction.

### Aromatic-Cationic Peptides

The disclosure relates to methods for decreasing the level of one or more of CRP, TNF-alpha, IL-6, ROS, cardiac troponin I, Nt-pro BNP, MLCL AT1, and ALCAT1 and/or increasing Taz1 expression and/or increasing mK ATP activity in a subject in need thereof, by administering aromatic-cationic peptides as disclosed herein. Reducing one or more of the level of CRP, TNF-alpha, IL-6, ROS, cardiac troponin I, Nt-pro BNP, MLCL AT1, and ALCAT1 and/or increasing Taz1 expression and/or increasing mK ATP activity may be useful for the treatment or prevention of heart failure and related conditions, reducing risk factors associated with heart failure, and/or reducing the likelihood (risk) or severity of heart failure in the subject.

The disclosure also relates to methods for preventing, ameliorating, or treating LV remodeling in a subject in need thereof, by administering aromatic-cationic peptides as disclosed herein. The subject may have an increased level of one or more of C-reactive protein, reactive oxygen species, interleukin-6, TNF-alpha, cardiac troponin I, Nt-pro BNP, MLCL AT1, and ALCAT1. A decrease in Nt-pro BNP and/or cardiac troponin I may be used as a biomarker to indicate the reduction or prevention of LV remodeling. The subject may have a decreased level of mK ATP activity or decreased expression of Taz1.

The disclosure also relates to methods for increasing the activity of mitochondrial ATP sensitive potassium channels (mK ATP) or expression of Taz1 in a subject in need thereof, by administering aromatic-cationic peptides as disclosed herein. Increasing the activity of mK ATP or expression of Taz1 may be useful for the treatment or prevention of heart failure and related conditions, reducing risk factors associated with heart failure, and/or reducing the likelihood (risk) or severity of heart failure in the subject.

The aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, more preferably about nine, and most preferably about six.

The amino acids of the aromatic-cationic peptides can be any amino acid. As used herein, the term "amino acid" is used to refer to any organic molecule that contains at least one amino group and at least one carboxyl group. Typically, at least one amino group is at the α position relative to a carboxyl group. The amino acids may be naturally occurring. Naturally occurring amino acids include, for example, the twenty most common levorotatory (L) amino acids normally found in mammalian proteins, *i.e.,* alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid includes hydroxyproline (Hyp).

The peptides optionally contain one or more non-naturally occurring amino acids. Optimally, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy *(i.e.,* alkoxy), amino, C₁-C₄ alkylamino and C₁-C₄ dialkylamino *(e.g.,* methylamino, dimethylamino), nitro, hydroxyl, halo *(i.e.,* fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, *e.g.* methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are suitably resistant or insensitive to common proteases. Examples of non-naturally occurring amino acids that are resistant or insensitive to proteases include the dextrorotatory (D-) form of any of the above-mentioned naturally occurring L-amino acids, as well as L- and/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, preferably less than four, more preferably less than three, and most preferably, less than two contiguous L-amino acids recognized by common proteases, irrespective of whether the amino acids are naturally or non-naturally occurring. Optimally, the peptide has only D-amino acids, and no L-amino acids. If the peptide contains protease sensitive sequences of amino acids, at least one of the amino acids is preferably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum numbers of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-D-Arg has one negatively charged amino acid *(i.e.,* Glu) and four positively charged amino acids *(i.e.,* two Arg residues, one Lys, and one His). Therefore, the above peptide has a net positive charge of three.

The aromatic-cationic peptides may have a relationship between the minimum number of net positive charges at physiological pH (pₘ) and the total number of amino acid residues (r) wherein 3pₘ is the largest number that is less than or equal to r + 1. The relationship between the minimum number of net positive charges (pₘ) and the total number of amino acid residues (r) may be as follows:

**TABLE 2. Amino acid number and net positive charges (3pₘ ≤ p+1)**

| **(r)** | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|
| **(pₘ)** | 1 | 1 | 2 | 2 | 2 | 3 | 3 | 3 | 4 | 4 | 4 | 5 | 5 | 5 | 6 | 6 | 6 | 7 |

The aromatic-cationic peptides may have a relationship between the minimum number of net positive charges (pₘ) and the total number of amino acid residues (r) wherein 2pₘ is the largest number that is less than or equal to r + 1. The relationship between the minimum number of net positive charges (pₘ) and the total number of amino acid residues (r) may be as follows:

**TABLE 3. Amino acid number and net positive charges (2pₘ ≤ p+1)**

| **(r)** | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|
| **(pₘ)** | 2 | 2 | 3 | 3 | 4 | 4 | 5 | 5 | 6 | 6 | 7 | 7 | 8 | 8 | 9 | 9 | 10 | 10 |

The minimum number of net positive charges (pₘ) and the total number of amino acid residues (r) may be equal. The peptides may have three or four amino acid residues and a minimum of one net positive charge, suitably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. The relationship between the minimum number of aromatic groups (a) and the total number of net positive charges (pₜ) may be as follows:

**TABLE 4. Aromatic groups and net positive charges (3a ≤ pₜ+1 or a= pₜ=1)**

| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| **(a)** | 1 | 1 | 1 | 1 | 2 | 2 | 2 | 3 | 3 | 3 | 4 | 4 | 4 | 5 | 5 | 5 | 6 | 6 | 6 | 7 |

The aromatic-cationic peptides may have a relationship between the minimum number of aromatic groups (a) and the total number of net positive charges (pₜ) wherein 2a is the largest number that is less than or equal to pₜ + 1. The relationship between the minimum number of aromatic amino acid residues (a) and the total number of net positive charges (pₜ) may be as follows:

**TABLE 5. Aromatic groups and net positive charges (2a ≤ pₜ+1 or a= pₜ=1)**

| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| **(a)** | 1 | 1 | 2 | 2 | 3 | 3 | 4 | 4 | 5 | 5 | 6 | 6 | 7 | 7 | 8 | 8 | 9 | 9 | 10 | 10 |

The number of aromatic groups (a) and the total number of net positive charges (pₜ) may be equal.

Carboxyl groups, especially the terminal carboxyl group of a C-terminal amino acid, are suitably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

The aromatic-cationic peptide may be a tripeptide having two net positive charges and at least one aromatic amino acid. The aromatic-cationic peptide may be a tripeptide having two net positive charges and two aromatic amino acids.

Aromatic-cationic peptides include, but are not limited to, the following peptide examples:

| **TABLE 6: EXEMPLARY PEPTIDES** |
|---|
| 2',6'-Dmp-D-Arg-2',6'-Dmt-Lys-NH₂ |
| 2',6'-Dmp-D-Arg-Phe-Lys-NH₂ |
| 2',6'-Dmt-D-Arg-PheOrn-NH₂ |
| 2',6'-Dmt-D-Arg-Phe-Ahp(2-aminoheptanoicacid)-NH₂ |
| 2',6'-Dmt-D-Arg-Phe-Lys-NH₂ |
| 2',6'-Dmt-D-Cit-PheLys-NH₂ |
| Ala-D-Phe-D-Arg-Tyr-Lys-D-Trp-His-D-Tyr-Gly-Phe |
| Arg-D-Leu-D-Tyr-Phe-Lys-Glu-D-Lys-Arg-D-Trp-Lys-D-Phe-Tyr-D-Arg-Gly |
| Asp-Arg-D-Phe-Cys-Phe-D-Arg-D-Lys-Tyr-Arg-D-Tyr-Trp-D-His-Tyr-D-Phe-Lys-Phe |
| Asp-D-Trp-Lys-Tyr-D-His-Phe-Arg-D-Gly-Lys-NH₂ |
| D-Arg-2',6'-Dmt-Lys-Phe-NH₂ |
| D-Glu-Asp-Lys-D-Arg-D-His-Phe-Phe-D-Val-Tyr-Arg-Tyr-D-Tyr-Arg-His-Phe-NH₂ |
| D-His-Glu-Lys-Tyr-D-Phe-Arg |
| D-His-Lys-Tyr-D-Phe-Glu-D-Asp-D-Asp-D-His-D-Lys-Arg-Trp-NH₂ |
| D-Tyr-Trp-Lys-NH₂ |
| Glu-Arg-D-Lys-Tyr-D-Val-Phe-D-His-Trp-Arg-D-Gly-Tyr-Arg-D-Met-NH₂ |
| Gly-Ala-Lys-Phe-D-Lys-Glu-Arg-Tyr-His-D-Arg-D-Arg-Asp-Tyr-Trp-D-His-Trp-His-D- Lys-Asp. |
| Gly-D-Phe-Lys-His-D-Arg-Tyr-NH₂ |
| His-Tyr-D-Arg-Trp-Lys-Phe-D-Asp-Ala-Arg-Cys-D-Tyr-His-Phe-D-Lys-Tyr-His-Ser-NH₂ |
| Lys-D-Arg-Tyr-NH₂ |
| Lys-D-Gln-Tyr-Arg-D-Phe-Trp-NH₂ |
| Lys-Trp-D-Tyr-Arg-Asn-Phe-Tyr-D-His-NH₂ |
| Met-Tyr-D-Arg-Phe-Arg-NH₂ |
| Met-Tyr-D-Lys-Phe-Arg |
| Phe-Arg-D-His-Asp |
| Phe-D-Arg-2',6'-Dmt-Lys-NH₂ |
| Phe-D-Arg-His |
| Phe-D-Arg-Lys-Trp-Tyr-D-Arg-Hi s |
| Phe-D-Arg-Phe-Lys-NH₂ |
| Phe-Phe-D-Tyr-Arg-Glu-Asp-D-Lys-Arg-D-Arg-His-Phe-NH₂ |
| Phe-Tyr-Lys-D-Arg-Trp-His-D-Lys-D-Lys-Glu-Arg-D-Tyr-Thr |
| Thr-Gly-Tyr-Arg-D-His-Phe-Trp-D-His-Lys |
| Thr-Tyr-Arg-D-Lys-Trp-Tyr-Glu-Asp-D-Lys-D-Arg-His-Phe-D-Tyr-Gly-Val-Ile-D-His- Arg-Tyr-Lys-NH2 |
| Trp-D-Lys-Tyr-Arg-NH₂ |
| Trp-Lys-Phe-D-Asp-Arg-Tyr-D-His-Lys |
| Tyr-Asp-D-Lys-Tyr-Phe-D-Lys-D-Arg-Phe-Pro-D-Tyr-His-Lys |
| Tyr-D-Arg-Phe-Lys-Glu-NH₂ |
| Tyr-D-Arg-Phe-Lys-NH₂ |
| Tyr-D-His-Phe-D-Arg-Asp-Lys-D-Arg-His-Trp-D-His-Phe |
| Tyr-His-D-Gly-Met |
| Val-D-Lys-His-Tyr-D-Phe-Ser-Tyr-Arg-NH₂ |

The peptides may have mu-opioid receptor agonist activity *(i.e.,* they activate the mu-opioid receptor). Peptides, which have mu-opioid receptor agonist activity, are typically those peptides that have a tyrosine residue or a tyrosine derivative at the N-terminus *(i.e.,* the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltryosine (Hmt).

A peptide that has mu-opioid receptor agonist activity may have the formula Tyr-D-Arg-Phe-Lys-NH₂. Tyr-D-Arg-Phe-Lys-NH₂ has a net positive charge of three, contributed by the amino acids tyrosine, arginine, and lysine and has two aromatic groups contributed by the amino acids phenylalanine and tyrosine. The tyrosine of Tyr-D-Arg-Phe-Lys-NH₂ can be a modified derivative of tyrosine such as in 2',6'-dimethyltyrosine to produce the compound having the formula 2',6'-Dmt-D-Arg-Phe-Lys-NH₂. 2',6'-Dmt-D-Arg-Phe-Lys-NH₂ has a molecular weight of 640 and carries a net three positive charge at physiological pH. 2',6'-Dmt-D-Arg-Phe-Lys-NH₂ readily penetrates the plasma membrane of several mammalian cell types in an energy-independent manner (Zhao et al., J. Pharmacol Exp Ther., 304:425-432, 2003).

Alternatively, in other instances, the aromatic-cationic peptide does not have mu-opioid receptor agonist activity. For example, during long-term treatment, such as in a chronic disease state or condition, the use of an aromatic-cationic peptide that activates the mu-opioid receptor may be contraindicated. In these instances, the potentially adverse or addictive effects of the aromatic-cationic peptide may preclude the use of an aromatic-cationic peptide that activates the mu-opioid receptor in the treatment regimen of a human patient or other mammal. Potential adverse effects may include sedation, constipation and respiratory depression. In such instances an aromatic-cationic peptide that does not activate the mu-opioid receptor may be an appropriate treatment. Peptides that do not have mu-opioid receptor agonist activity generally do not have a tyrosine residue or a derivative of tyrosine at the N-terminus *(i.e.,* amino acid position 1). The amino acid at the N-terminus can be any naturally occurring or non-naturally occurring amino acid other than tyrosine. The amino acid at the N-terminus may be phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

An example of an aromatic-cationic peptide that does not have mu-opioid receptor agonist activity has the formula Phe-D-Arg-Phe-Lys-NH₂. Alternatively, the N-terminal phenylalanine can be a derivative of phenylalanine such as 2',6'-dimethylphenylalanine (2'6'-Dmp). Tyr-D-Arg-Phe-Lys-NH₂ containing 2',6'-dimethylphenylalanine at amino acid position 1 has the formula 2',6'-Dmp-D-Arg-Phe-Lys-NH₂. The amino acid sequence of 2',6'-Dmt-D-Arg-Phe-Lys-NH₂ may be rearranged such that Dmt is not at the N-terminus. An example of such an aromatic-cationic peptide that does not have mu-opioid receptor agonist activity has the formula D-Arg-2',6'-Dmt-Lys-Phe-NH₂.

Suitable substitution variants of the peptides listed herein include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group are generally more likely to alter the characteristics of the original peptide.

Examples of peptides that activate mu-opioid receptors include, but are not limited to, the aromatic-cationic peptides shown in Table 7.

**TABLE 7. Peptide Analogs with Mu-Opioid Activity**

| **Amino Acid Position 1** | **Amino Acid Position 2** | **Amino Acid Position 3** | **Amino Acid Position 4** | **C-Terminal Modification** |
|---|---|---|---|---|
| Tyr | D-Arg | Phe | Lys | **NH₂** |
| Tyr | D-Arg | Phe | Orn | NH₂ |
| Tyr | D-Arg | Phe | Dab | NH₂ |
| Tyr | D-Arg | Phe | Dap | NH₂ |
| 2',6'Dmt | D-Arg | Phe | Lys | NH₂ |
| 2',6'Dmt | D-Arg | Phe | Lys-NH(CH₂)₂-NH-dns | NH₂ |
| 2',6'Dmt | D-Arg | Phe | Lys-NH(CH₂)₂-NH-atn | NH₂ |
| 2',6'Dmt | D-Arg | Phe | dnsLys | NH₂ |
| 2',6'Dmt | D-Cit | Phe | Lys | NH₂ |
| 2',6'Dmt | D-Cit | Phe | Ahp | NH₂ |
| 2',6'Dmt | D-Arg | Phe | Orn | NH₂ |
| 2',6'Dmt | D-Arg | Phe | Dab | NH₂ |
| 2',6'Dmt | D-Arg | Phe | Dap | NH₂ |
| 2',6'Dmt | D-Arg | Phe | Ahp(2-aminoheptanoic acid) | NH₂ |
| Bio-2',6'Dmt | D-Arg | Phe | Lys | NH₂ |
| 3',5'Dmt | D-Arg | Phe | Lys | NH₂ |
| 3',5'Dmt | D-Arg | Phe | Orn | NH₂ |
| 3',5'Dmt | D-Arg | Phe | Dab | NH₂ |
| 3',5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2',6'Dmt | D-Arg | Tyr | Lys | NH₂ |
| 2',6'Dmt | D-Arg | Tyr | Orn | NH₂ |
| 2',6'Dmt | D-Arg | Tyr | Dab | NH₂ |
| 2',6'Dmt | D-Arg | Tyr | Dap | NH₂ |
| 2',6'Dmt | D-Arg | 2'6'Dmt | Lys | NH₂ |
| 2',6'Dmt | D-Arg | 2'6'Dmt | Orn | NH₂ |
| 2',6'Dmt | D-Arg | 2'6'Dmt | Dab | NH₂ |
| 2',6'Dmt | D-Arg | 2'6'Dmt | Dap | NH₂ |
| 3',5'Dmt | D-Arg | 3'5'Dmt | Arg | NH₂ |
| 3',5'Dmt | D-Arg | 3'5'Dmt | Lys | NH₂ |
| 3',5'Dmt | D-Arg | 3'5'Dmt | Orn | NH₂ |
| 3',5'Dmt | D-Arg | 3'5'Dmt | Dab | NH₂ |
| Tyr | D-Lys | Phe | Dap | NH₂ |
| Tyr | D-Lys | Phe | Arg | NH₂ |
| Tyr | D-Lys | Phe | Lys | NH₂ |
| Tyr | D-Lys | Phe | Orn | NH₂ |
| 2',6'Dmt | D-Lys | Phe | Dab | NH₂ |
| 2',6'Dmt | D-Lys | Phe | Dap | NH₂ |
| 2',6'Dmt | D-Lys | Phe | Arg | NH₂ |
| 2',6'Dmt | D-Lys | Phe | Lys | NH₂ |
| 3',5'Dmt | D-Lys | Phe | Orn | NH₂ |
| 3',5'Dmt | D-Lys | Phe | Dab | NH₂ |
| 3',5'Dmt | D-Lys | Phe | Dap | NH₂ |
| 3',5'Dmt | D-Lys | Phe | Arg | NH₂ |
| Tyr | D-Lys | Tyr | Lys | NH₂ |
| Tyr | D-Lys | Tyr | Orn | NH₂ |
| Tyr | D-Lys | Tyr | Dab | NH₂ |
| Tyr | D-Lys | Tyr | Dap | NH₂ |
| 2',6'Dmt | D-Lys | Tyr | Lys | NH₂ |
| 2',6'Dmt | D-Lys | Tyr | Orn | NH₂ |
| 2',6'Dmt | D-Lys | Tyr | Dab | NH₂ |
| 2',6'Dmt | D-Lys | Tyr | Dap | NH₂ |
| 2',6'Dmt | D-Lys | 2'6'Dmt | Lys | NH₂ |
| 2',6'Dmt | D-Lys | 2'6'Dmt | Orn | NH₂ |
| 2',6'Dmt | D-Lys | 2'6'Dmt | Dab | NH₂ |
| 2',6'Dmt | D-Lys | 2'6'Dmt | Dap | NH₂ |
| 2',6'Dmt | D-Arg | Phe | dnsDap | NH₂ |
| 2',6'Dmt | D-Arg | Phe | atnDap | NH₂ |
| 3',5'Dmt | D-Lys | 3'5'Dmt | Lys | NH₂ |
| 3',5'Dmt | D-Lys | 3'5'Dmt | Orn | NH₂ |
| 3',5'Dmt | D-Lys | 3'5'Dmt | Dab | NH₂ |
| 3',5'Dmt | D-Lys | 3'5'Dmt | Dap | NH₂ |
| Tyr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2',6'Dmt | D-Arg | Phe | Arg | NH₂ |
| 2',6'Dmt | D-Lys | Phe | Arg | NH₂ |
| 2',6'Dmt | D-Orn | Phe | Arg | NH₂ |
| 2',6'Dmt | D-Dab | Phe | Arg | NH₂ |
| 3',5'Dmt | D-Dap | Phe | Arg | NH₂ |
| 3',5'Dmt | D-Arg | Phe | Arg | NH₂ |
| 3',5'Dmt | D-Lys | Phe | Arg | NH₂ |
| 3',5'Dmt | D-Orn | Phe | Arg | NH₂ |
| Tyr | D-Lys | Tyr | Arg | NH₂ |
| Tyr | D-Orn | Tyr | Arg | NH₂ |
| Tyr | D-Dab | Tyr | Arg | NH₂ |
| Tyr | D-Dap | Tyr | Arg | NH₂ |
| 2',6'Dmt | D-Arg | 2'6'Dmt | Arg | NH₂ |
| 2',6'Dmt | D-Lys | 2'6'Dmt | Arg | NH₂ |
| 2',6'Dmt | D-Orn | 2'6'Dmt | Arg | NH₂ |
| 2',6'Dmt | D-Dab | 2'6'Dmt | Arg | NH₂ |
| 3',5'Dmt | D-Dap | 3'5'Dmt | Arg | NH₂ |
| 3',5'Dmt | D-Arg | 3'5'Dmt | Arg | NH₂ |
| 3',5'Dmt | D-Lys | 3'5'Dmt | Arg | NH₂ |
| 3',5'Dmt | D-Orn | 3'5'Dmt | Arg | NH₂ |
| Mmt | D-Arg | Phe | Lys | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-Arg | Phe | Orn | NH₂ |
| Tmt | D-Arg | Phe | Dab | NH₂ |
| Tmt | D-Arg | Phe | Dap | NH₂ |
| Hmt | D-Arg | Phe | Lys | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Lys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lys | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |

| | | | | |
|---|---|---|---|---|
| Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = β-dansyl-L-α,β-diaminopropionic acid atnDap = β-anthraniloyl-L-α,β-diaminopropionic acid Bio = biotin | | | | |

Examples of peptides that do not activate mu-opioid receptors include, but are not limited to, the aromatic-cationic peptides shown in Table 8.

**TABLE 8. Peptide Analogs Lacking Mu-Opioid Activity**

| **Amino Acid Position 1** | **Amino Acid Position 2** | **Amino Acid Position 3** | **Amino Acid Position 4** | **C-Terminal Modification** |
|---|---|---|---|---|
| D-Arg | Dmt | Lys | Phe | NH₂ |
| D-Arg | Dmt | Phe | Lys | NH₂ |
| D-Arg | Phe | Lys | Dmt | NH₂ |
| D-Arg | Phe | Dmt | Lys | NH₂ |
| D-Arg | Lys | Dmt | Phe | NH₂ |
| D-Arg | Lys | Phe | Dmt | NH₂ |
| Phe | Lys | Dmt | D-Arg | NH₂ |
| Phe | Lys | D-Arg | Dmt | NH₂ |
| Phe | D-Arg | Phe | Lys | NH₂ |
| Phe | D-Arg | Dmt | Lys | NH₂ |
| Phe | D-Arg | Lys | Dmt | NH₂ |
| Phe | Dmt | D-Arg | Lys | NH₂ |
| Phe | Dmt | Lys | D-Arg | NH₂ |
| Lys | Phe | D-Arg | Dmt | NH₂ |
| Lys | Phe | Dmt | D-Arg | NH₂ |
| Lys | Dmt | D-Arg | Phe | NH₂ |
| Lys | Dmt | Phe | D-Arg | NH₂ |
| Lys | D-Arg | Phe | Dmt | NH₂ |
| Lys | D-Arg | Dmt | Phe | NH₂ |
| D-Arg | Dmt | D-Arg | Phe | NH₂ |
| D-Arg | Dmt | D-Arg | Dmt | NH₂ |
| D-Arg | Dmt | D-Arg | Tyr | NH₂ |
| D-Arg | Dmt | D-Arg | Trp | NH₂ |
| Trp | D-Arg | Phe | Lys | NH₂ |
| Trp | D-Arg | Tyr | Lys | NH₂ |
| Trp | D-Arg | Trp | Lys | NH₂ |
| Trp | D-Arg | Dmt | Lys | NH₂ |
| D-Arg | Tip | Lys | Phe | NH₂ |
| D-Arg | Tip | Phe | Lys | NH₂ |
| D-Arg | Tip | Lys | Dmt | NH₂ |
| D-Arg | Tip | Dmt | Lys | NH₂ |
| D-Arg | Lys | Tip | Phe | NH₂ |
| D-Arg | Lys | Tip | Dmt | NH₂ |
| Cha | D-Arg | Phe | Lys | NH₂ |
| Ala | D-Arg | Phe | Lys | NH₂ |

| | | | | |
|---|---|---|---|---|
| Cha = cyclohexyl alanine | | | | |

The amino acids of the peptides shown in Table 7 and 8 may be in either the L- or the D- configuration.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc., New York (1997).

### Prophylactic and Therapeutic Uses of Aromatic-Cationic Peptides

*General.* The aromatic-cationic peptides described herein are useful to prevent or treat disease. Specifically, the disclosure provides for both prophylactic and therapeutic methods of treating a subject having or suspected of having one or more of an elevated CRP level, *e.g.,* as determined by hsCRP assay, an elevated TNF-alpha level, an elevated IL-6 level, or an elevated ROS level, an elevated cardiac troponin I level, an elevated Nt-pro BNP level, an elevated MLCL AT1 expression level, an elevated ALCAT 1 expression level, and/or a decrease in mK ATP activity and/or decrease in Taz1 expression. For example, the disclosure provides for both prophylactic and therapeutic methods of treating a subject having or at risk of (susceptible to) heart failure, and having or suspected of having an elevated CRP level, *e.g.,* as determined by hsCRP, an elevated TNF-alpha level, an elevated IL-6 level, an elevated ROS level, an elevated cardiac troponin I level, an elevated Nt-pro BNP level, an elevated MLCL AT1 expression level, an elevated ALCAT 1 expression level, and/or a decrease in mK ATP activity and/or decrease in Taz1 expression. Accordingly, the disclosure relates to methods of preventing and/or treating heart failure in a subject by administering an effective amount of an aromatic-cationic peptide to a subject in need thereof to reduce or normalize one or more of the CRP level, the TNF-alpha level, the IL-6 level, the ROS level, the cardiac troponin I level, MLCL AT1 expression level, the ALCAT 1 expression level, and/or Nt-pro BNP, and/or to increase or normalize the Taz1 expression level and/or the mK ATP activity of the subject.

Additionally, the disclosure provides for both prophylactic and therapeutic methods of treating a subject at risk of LV remodeling, or suffering from LV remodeling, e.g., due to elevated CRP, TNF-alpha, IL-6, ROS, cardiac troponin I, MLCL AT1, or ALCAT 1 and/or a decrease is mK ATP activity and/or decrease in Taz1 expression. Accordingly, the present methods provide for the prevention and/or treatment of LV remodeling in a subject by administering an effective amount of an aromatic-cationic peptide to a subject in need thereof.

Elevation of one or more of CRP level, TNF-alpha level, IL-6 level, or ROS level, or Nt-pro BNP level, or cardiac troponin I level, or MLCL AT1 expression, or ALCAT 1 expression serve to indicate future vascular events in subjects without any previous history of cardiovascular disease. The decreased activity of mK ATP or decreased Taz 1 expression may serve to indicate future vascular events in subjects without any previous history of cardiovascular disease. The determination of one or more of CRP level, TNF-alpha level, IL-6 level, or ROS, or Nt-pro BNP level, or cardiac troponin I level, or MLCL AT1 expression, or ALCAT 1 expression, mK ATP activity, or Taz1 expression may enhance risk assessment and therapeutic outcomes in primary cardiovascular disease prevention. One or more of CRP level, TNF-alpha level, IL-6 level, or ROS level, or Nt-pro BNP level, or cardiac troponin I level, or MLCL AT1 expression, or ALCAT 1 expression, or Taz1 expression, or mK ATP activity may serve as an independent marker of for evaluating the possibility of recurrent cardiac events, such as myocardial infarction or restenosis, e.g., after percutaneous coronary intervention. One or more of CRP level, TNF-alpha level, IL-6 level, or ROS level, or Nt-pro BNP level, or cardiac troponin I level, or mK ATP activity, or MLCL AT1 expression, or ALCAT 1 expression, or Taz1 expression may be used in cardiac risk stratification and assessment, and are prognostic factors in conditions such as acute coronary syndrome, stroke peripheral artery disease, and post myocardial infarction complication such as cardiac failure. One or more of CRP level, TNF-alpha level, IL-6 level, and ROS level, or Nt-pro BNP level, or cardiac troponin I level or mK ATP activity, or MLCL AT1 expression, or ALCAT 1 expression, or Taz1 expression may be used to determine the probability of recurrence of cardiac events in patients with stable coronary heart disease and/or acute coronary syndrome. One or more of CRP level, TNF-alpha level, IL-6 level, or ROS level, or Nt-pro BNP level, or cardiac troponin I level or mK ATP activity, or MLCL AT1 expression, or ALCAT 1 expression, or Taz1 expression may be a predictor of early complications and late clinical stenosis and mortality in subjects undergoing percutaneous coronary interventions. One or more of CRP level, TNF-alpha level, IL-6 level, or ROS level, or Nt-pro BNP level, or cardiac troponin I level, or mK ATP activity, or MLCL AT1 expression, or ALCAT 1 expression, or Taz1 expression may add prognostic value in acute coronary syndrome, stable angina, unstable angina, and acute myocardial infarction. Measuring one or more of CRP level, TNF-alpha level, IL-6 level, or ROS level, or Nt-pro BNP level, or cardiac troponin I level, or mK ATP activity, or MLCL AT1 expression, or ALCAT 1 expression, or Taz1 expression may add prognostic value in LV remodeling.

Accordingly, therapeutic and/or prophylactic treatment of subjects having one or more of elevated CRP level, TNF-alpha level, IL-6 level, ROS level, cardiac troponin I level, or MLCL AT1 expression, and/or ALCAT 1 expression with an aromatic cationic peptide as disclosed herein, such as, *e.g.,* D-Arg-2',6'Dmt- Lys-Phe-NH2 or a pharmaceutically acceptable salt thereof, such as acetate or trifluoroacetate salt, may reduce one or more of the elevated CRP level, the TNF-alpha level, the IL-6 level, ROS level, the cardiac troponin I level, the MLCL AT1 expression, and/or the ALCAT 1 expression thereby reducing the risk of any of the cardiac, stenotic/vascular events, e.g., LV remodeling, described above. Additionally, or alternatively, therapeutic and/or prophylactic treatment of subjects having decreased mK ATP activity and/or decreased Taz1 expression with an aromatic cationic peptide as disclosed herein, such as, *e.g.,* D-Arg-2',6' Dmt- Lys-Phe-NH₂ or a pharmaceutically acceptable salt thereof, such as acetate or trifluoroacetate salt, may increase mK ATP activity and/or Taz1 expression, thereby reducing the risk of any of the cardiac, stenotic/vascular events, *e.g.,* LV remodeling, described above. One or more of the CRP level, the TNF-alpha level, the IL-6 level, the ROS level, Nt-pro BNP level, or cardiac troponin I level, or Nt-pro BNP level, or mK ATP activity, or MLCL AT1 expression, or ALCAT 1 expression, or Taz1 expression may be normalized after treatment with the aromatic-cationic peptide.

*Therapeutic Methods.* The following discussion is presented by way of example only, and is not intended to limit the disclosed methods and compositions to a specific disease or disease state. It is understood that lowering one or more of a subject's CRP level, or TNF-alpha level, or IL-6 level, or ROS level, cardiac troponin I level, MLCL AT1 expression, or ALCAT 1 expression and/or increasing mK ATP activity and/or increasing Taz1 expression in the subject will reduce the risk of any number of negative cardiac, stenotic or vascular events, e.g., LV remodeling. The disclosure also includes methods for treating LV remodeling in a subject having or suspected of having an elevated CRP level, or TNF-alpha level, or IL-6 level, or ROS level, or cardiac troponin I level, or MLCL AT1 expression, or ALCAT 1 expression and/or decreased mK ATP activity and/or decreased Taz1 expression for therapeutic purposes. In therapeutic applications, compositions or medicaments are administered to a subject suspected of, or already suffering from such a disease in an amount sufficient to cure, or at least partially arrest, the symptoms of the disease, including its complications and intermediate pathological phenotypes in development of the disease. The disclosure also inlcudes methods of treating an individual having or suspected of having an elevated CRP level, TNF-alpha level, IL-6 level, ROS level, cardiac troponin I level, MLCL AT1 expression, and/or ALCAT 1 expression and/or decreased mK ATP and/or decreased Taz1 expression afflicted with heart failure or LV remodeling.

Subjects suffering from heart failure can be identified by any or a combination of diagnostic or prognostic assays known in the art. For example, typical symptoms of heart failure include shortness of breath (dyspnea), fatigue, weakness, difficulty breathing when lying flat, and swelling of the legs, ankles or abdomen (edema). The subject may also exhibit elevated CRP level, TNF-alpha level, IL-6 level, ROS level, NT-pro BNP level, cardiac troponin I level, MLCL AT1 expression, and/or ALCAT 1 expression and/or decreased mK ATP and/or decreased Taz1 expression. The subject may also be suffering from other disorders including coronary artery disease, systemic hypertension, cardiomyopathy or myocarditis, congenital heart disease, abnormal heart valves or valvular heart disease, severe lung disease, diabetes, severe anemia hyperthyroidism, arrhythmia or dysrhythmia and myocardial infarction. The primary signs of congestive heart failure are cardiomegaly (enlarged heart), tachypnea (rapid breathing; occurs in the case of left side failure) and hepatomegaly (enlarged liver; occurs in the case of right side failure). Acute myocardial infarction ("AMI") due to obstruction of a coronary artery is a common initiating event that can lead ultimately to heart failure. However, a subject that has AMI does not necessarily develop heart failure. Likewise, subjects that suffer from heart failure did not necessarily suffer from an AMI.

Subjects suffering from LV remodeling can be identified by any or a combination of diagnostic or prognostic assays known in the art. For example, typical symptoms of LV remodeling include decreased LV stroke volume, reduced LV ejection fraction, poor fractional shortening, increased infarct expansion, poor hemodynamics, increased scar formation in LV myocardium, and increased lung volumes. Symptoms of LV remodeling also include symptoms associated with heart failure such as, e.g., shortness of breath, fatigue, and swelling of the extremities. A "therapeutically effective amount" of aromatic-cationic peptides may include levels in which the physiological effects of elevated CRP level, TNF-alpha level, IL-6 level, ROS level, cardiac troponin I level, MLCL AT1 expression, and/or ALCAT 1 expression are, at a minimum, ameliorated. Additionally, or alternatively, a therapeutically effective amount of the aromatic-cationic peptides may include levels in which the physiological effects of decreased mK ATP levels and/or decreased Taz1 expression are, at a minimum, ameliorated. Additionally, or alternatively, a therapeutically effective amount of the aromatic-cationic peptides may include levels in which the physiological effects of LV remodeling are, at a minimum, ameliorated.

*Prophylactic Methods.* The disclosure also includes a method for preventing or reducing the likelihood or severity of heart failure in a subject having or suspected of having one or more of an elevated CRP level, TNF-alpha level, or IL-6 level, or ROS level, cardiac troponin I level, MLCL AT1 expression, and/or ALCAT 1 expression and/or decreased mK ATP and/or decreased Taz1 expression by administering to the subject an aromatic-cationic peptide that reduces *(e.g.,* normalizes) one or more of the CRP level, TNF-alpha level, IL-6 level, ROS level, cardiac troponin I level, MLCL AT1 expression, ALCAT 1 expression and/or increases *(e.g.,* normalizes) mK ATP and/or increases Taz1 expression. Subjects at risk for heart failure can be identified by, *e.g.,* any or a combination of diagnostic or prognostic assays as described herein. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the disease or disorder, such that the disease or disorder is prevented or, alternatively, delayed in its progression. The appropriate compound can be determined based on screening assays described above.

Subjects diagnosed with or at risk for heart failure may exhibit one or more of the following non-limiting risk factors: high blood pressure; coronary artery disease; heart attack; irregular heartbeats; diabetes; some diabetes medications *(e.g.,* rosiglitazone and pioglitazone have been found to increase the risk of heart failure); sleep apnea; congenital heart defects; viral infection; alcohol use; obesity, lifestyle *(e.g.,* smoking, sedentary lifestyle), high cholesterol, family history, stress, and kidney conditions.

### Improvement of Left Ventricular Function

Patients with HF often have elevated levels of pro-inflammatory cytokines and chemokines, compounds that are involved in adverse left ventricular (LV) remodeling, neurohormonal activation, impaired autonomic and vascular tone, and progression of coronary atherosclerosis (Sola et al. "Atorvastatin Improves Left Ventricular Systolic Function and Serum Markers of Inflammation in Nonischemic Heart Failure." J. Am. Coll. Cardiol. 47(2): 332-337 (2006)). Higher levels of these inflammatory markers, including TNF-α, IL-6, and CRP, are also associated with adverse cardiovascular morbidity and mortality. The reduction of pro-inflammatory cytokines, *e.g.,* TNF-alpha, IL-6, and CRP, by aromatic-cationic peptides, *e.g.,* D-Arg-2',6'-Dmt-Lys-Phe-NH₂, may decrease LV remodeling.

The reduction of pro-inflammatory cytokines, *e.g.,* TNF-alpha, IL-6, and CRP, by aromatic-cationic peptides, *e.g.,* D-Arg-2',6'-Dmt-Lys-Phe-NH₂, may improve LV function in subjects at risk for HF, or suffering from HF, or other stenotic or vascular events. Improvement in LV function (as compared to untreated subjects) may be measured by decreased LV stroke volume, increased LV ejection fraction, improved fractional shortening, decreased infarct expansion, good hemodynamics, decreased scar formation in LV myocardium, decreased lung volumes, or a combination thereof.

In acute myocardial infarction and HF, ROS is generated in the ischemic myocardium, especially after reperfusion. ROS directly injure the cell membrane and cause cell death. ROS also stimulates signal transduction to elaborate inflammatory cytokines, e.g., TNF-alpha, IL-1 beta and -6, in the ischemic region and surrounding myocardium as a host reaction. Inflammatory cytokines also regulate cell survival and cell death in the chain reaction with ROS.

The reduction of ROS, by aromatic-cationic peptides, *e.g.,* D-Arg-2',6'-Dmt-Lys-Phe-NH₂, may improve LV function after myocardial infarction or in subjects at risk for HF. Improvement in LV function (as compared to untreated subjects) may be measured by decreased LV stroke volume, increased LV ejection fraction, improved fractional shortening, decreased infarct expansion, good hemodynamics, decreased scar formation in LV myocardium, decreased lung volumes, or a combination thereof.

In acute myocardial infarction and HF, cardiac troponin I is generated in the ischemic myocardium. Cardiac troponin I can lead to the increase in cardiac troponin autoantibodies. Increased cardiac troponin autoantibodies can increase cardiac inflammation and increase expression of inflammatory cytokines.

The reduction of cardiac troponin I by aromatic-cationic peptides, *e.g.,* D-Arg-2',6'-Dmt-Lys-Phe-NH₂, may improve LV function after myocardial infarction or in subjects at risk for HF. Improvement in LV function (as compared to untreated subjects) may be measured by decreased LV stroke volume, increased LV ejection fraction, improved fractional shortening, decreased infarct expansion, good hemodynamics, decreased scar formation in LV myocardium, decreased lung volumes, or a combination thereof.

In acute myocardial infarction and HF, a decrease in the reduced form of NADPH leads to decreased mK ATP activity. The decrease in mK ATP activity leads to ionic deregulation in mitochondrial environment with subsequent matrix contraction and reduced ATP production.

The increase in mK ATP activity by aromatic-cationic peptides, *e.g.,* D-Arg-2',6'-Dmt-Lys-Phe-NH₂, may improve LV function after myocardial infarction or in subjects at risk for HF. Improvement in LV function (as compared to untreated subjects) may be measured by decreased LV stroke volume, increased LV ejection fraction, improved fractional shortening, decreased infarct expansion, good hemodynamics, decreased scar formation in LV myocardium, decreased lung volumes, or a combination thereof.

### Determination of the Biological Effect of the Aromatic-Cationic Peptide-Based Therapeutic

Suitable *in vitro* or *in vivo* assays may be performed to determine the effect of a specific aromatic-cationic peptide-based therapeutic and whether its administration is indicated for treatment. *In vitro* assays can be performed with representative animal models, to determine if a given aromatic-cationic peptide-based therapeutic exerts the desired effect in reducing one or more of CRP levels, TNF-alpha levels, IL-6 levels, ROS levels, cardiac troponin I level, MLCL AT1 expression, and/or ALCAT 1 expression and/or increasing mK ATP activity and/or increasing Taz1 expression, thereby preventing or treating heart failure or LV remodeling. Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art can be used prior to administration to human subjects.

HF has been induced in different species with volume overload, pressure overload, fast pacing, myocardial ischemia, cardiotoxic drugs, or genetically modified models. Models using pressure overload have been most commonly used. Hypertension is associated with an increased risk for the development of HF. In one mouse model, angiotensin II (Ang II) increases blood pressure and induces cardiomyocyte hypertrophy, increased cardiac fibrosis, and impaired cardiomyocyte relaxation. Infusion of angiotensin to mice by mini osmotic pump increases systolic and diastolic blood pressure, increases heart weight and left ventricular thickness (LVMI), and impaired myocardial performance index (MPI). CRP levels, TNF-alpha levels, IL-6 levels, ROS levels, and/or cardiac troponin I levels, MLCL AT1, ALCAT1, and/or Taz1 expression levels, and/or mK ATP activity are monitored at various time points before, during and after HF induction.

In a second illustrative mouse model, sustained high level expression of Gaq can lead to marked myocyte apoptosis, resulting in cardiac hypertrophy and heart failure by 16 weeks of age (D'Angelo *et al.,* 1998). The β-adrenergic receptors (βARs) are primarily coupled to the heterotrimeric G protein, Gs, to stimulate adenylyl cyclase activity. This association generates intracellular cAMP and protein kinase A activation, which regulate cardiac contractility and heart rate. Overexpression of Gαq leads to decreased responsiveness to β-adrenergic agonists and results in HF. CRP levels, TNF-alpha levels, IL-6 levels, ROS levels, and/or cardiac troponin I levels, MLCL AT1, ALCAT1, and/or Taz1 expression levels, and/or mK ATP activity are monitored at various time points before, during and after HF induction.

Experimental constriction of the aorta by surgical ligation is also widely used as a model of HF. Transaortic constriction (TAC) results in pressure overload induced HF, with increase in left ventricular (LV) mass. TAC is performed as described by Tamavski O *et al.* (2004) using a 7-0 silk double-knot suture to constrict the ascending aorta. After TAC, mice develop HF within a period of 4 weeks. CRP levels, TNF-alpha levels, IL-6 levels, ROS levels, and/or cardiac troponin I levels, MLCL AT1, ALCAT1, and/or Taz1 expression levels, and/or mK ATP activity are monitored at various time points before, during and after HF induction.

### Prophylactic and Therapeutic Uses for CRP

As noted above, numerous studies have linked CRP levels, as determined in high sensitivity assays, to cardiac risk. One exemplary risk assessment scale is shown in Table 9:

| **Table 9: hsCRP levels correlated with cardiovascular risk** | |
|---|---|
| *hsCRP measurement* | *Cardiovascular risk* |
| <1 mg/L | low risk |
| 1-3 mg/L | intermediate risk |
| 3-10 mg/L | high risk |
| >10 mg/L | unspecific elevation |

A normal, normalized or control CRP level may be less than about 1 mg/L. A control, normal or normalized CRP level may be between about 1 and 3 mg/L.

A lower CRP level may be determined by hsCRP assay. A reduced or lowered CRP level may be from a high cardiac risk level *(e.g.,* 3 mg/L or greater) to a lower risk level *(e.g.,* 1-3 mg/L or less than 1 mg/L). Reducing CRP levels may be a reduction by about 1 mg/L, about 2 mg/L, about 3 mg/L, about 4 mg/L, about 5 mg/L, about 6 mg/L, about 7 mg/L , about 8 mg/L or about 9 mg/L.

### Prophylactic and Therapeutic Uses for ROS

A normal, normalized, or control ROS level may be less than about 3 RLU x 10⁵/ml. A normal, normalized, or control ROS level may be between about 3 RLU x 10⁵/ml to about 13 RLU x 10⁵/ml.

Reducing ROS levels may be a reduction between about 1 RLU x 10⁵/ml to about 5 RLU x 10⁵/ml, between about 5 RLU x 10⁵/ml to about 10 RLU x 10⁵/ml, between about 10 RLU x 10⁵/ml to about 15 RLU x 10⁵/ml, between about 15 RLU x 10⁵/ml to about 20 RLU x 10⁵/ml, or between about 20 RLU x 10⁵/ml to about 25 RLU x 10⁵/ml.

### Prophylactic and Therapeutic Uses for IL-6

A normal, normalized, or control IL-6 level may be less than about 10 pg/ml. In some embodiments, a normal, normalized, or control IL-6 level is between about 5 pg/ml to about 13 pg/ml.

Reducing IL-6 levels may be a reduction between about 1 pg/ml to about 5 pg/ml, between about 5 pg/ml to about 10 pg/ml, between about 10 pg/ml to about 15 pg/ml, or between about 15 pg/ml to about 20 pg/ml.

### Prophylactic and Therapeutic Uses for TNF-alpha

A normal, normalized, or control TNF-alpha level may be less than about 1.5 pg/ml. A normal, normalized, or control TNF-alpha level may be between about 1 pg/ml to about 2 pg/ml.

Reducing TNF-alpha levels may be a reduction about 1 pg/ml, or about 2 pg/ml, or about 3 pg/ml, or about 4 pg/ml, or 5 pg/ml.

### Prophylactic and Therapeutic Uses for MLCL AT1, AL CAT1, and Taz1

*Therapeutic Methods:* The following discussion is presented by way of example only, and is not intended to limit the disclosed methods and compositions to a specific disease or disease state. It is understood that lowering the expression of MLCL AT1 or ALCAT1 and/or raising the expression Taz1 in a subject in need thereof will reduce the risk of any number of negative cardiac, stenotic or vascular events. One aspect of the present technology includes methods of treating heart failure in a subject having or suspected of having an elevated MLCL AT1 or ALCAT1 expression and/or lowered Taz1 expression for therapeutic purposes. In therapeutic applications, compositions or medicaments are administered to a subject suspected of, or already suffering from such a disease in an amount sufficient to cure, or at least partially arrest, the symptoms of the disease, including its complications and intermediate pathological phenotypes in development of the disease. The present technology disclsoses methods of treating an individual having or suspected of having an elevated MLCL AT1 or ALCAT1 expression level afflicted with heart failure. Alternatively, or additionally, the present technology discloses methods of treating an individual having or suspected of having an decreased Taz1 expression afflicted with heart failure.

Subjects suffering from heart failure can be identified by any or a combination of diagnostic or prognostic assays known in the art. For example, typical symptoms of heart failure include shortness of breath (dyspnea), fatigue, weakness, difficulty breathing when lying flat, and swelling of the legs, ankles or abdomen (edema). The subject may also exhibit elevated MLCL AT1 or ALCAT1 expression and/or lowered Taz1 expression. The subject may also be suffering from other disorders including coronary artery disease, systemic hypertension, cardiomyopathy or myocarditis, congenital heart disease, abnormal heart valves or valvular heart disease, severe lung disease, diabetes, severe anemia hyperthyroidism, arrhythmia or dysrhythmia and myocardial infarction. The primary signs of congestive heart failure are cardiomegaly (enlarged heart), tachypnea (rapid breathing; occurs in the case of left side failure) and hepatomegaly (enlarged liver; occurs in the case of right side failure). Acute myocardial infarction ("AMI") due to obstruction of a coronary artery is a common initiating event that can lead ultimately to heart failure. However, a subject that has AMI does not necessarily develop heart failure. Likewise, subjects that suffer from heart failure did not necessarily suffer from an AMI.

*Prophylactic Methods:* The disclosure relates to a method for preventing heart failure in a subject having or suspected of having one or more of an elevated MLCL AT1 or ALCAT1 expression and/or decreased Taz1 expression, by administering to the subject an aromatic-cationic peptide that normalizes one or more of the MLCL AT1, ALCAT1, or Taz1 expression levels. Thus, in one aspect of the invention, there is provided a composition for use in preventing or reducing the risk of heart failure in a human subject in need thereof, wherein the subject has higher than a control or "normal" level of ALCAT 1, wherein the composition comprises a therapeutically effective amount of the peptide D-Arg-2',6'-Dmt-Lys-Phe-NH₂ or a pharmaceutically acceptable salt thereof. Subjects at risk for heart failure can be identified by, *e.g.,* any or a combination of diagnostic or prognostic assays as described herein. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the disease or disorder, such that the disease or disorder is prevented or, alternatively, delayed in its progression. The appropriate compound can be determined based on screening assays described above.

Subjects diagnosed with or at risk for heart failure may exhibit one or more of the following non-limiting risk factors: high blood pressure; coronary artery disease; heart attack; irregular heartbeats; diabetes; some diabetes medications *(e.g.,* rosiglitazone and pioglitazone have been found to increase the risk of heart failure); sleep apnea; congenital heart defects; viral infection; alcohol use; obesity, lifestyle *(e.g.,* smoking, sedentary lifestyle), high cholesterol, family history, stress, and kidney conditions.

### C-reactive protein as a biomarker for peptide dosage.

C-reactive protein levels may be used to determine the dosage of aromatic-cationic peptide *(e.g.,* D-Arg-2',6'-Dmt-Lys-Phe-NH₂) to administer to a subject. For example, if the level of C-reactive protein in a subject is less than about 1 mg/L, no or low levels of an aromatic cationic peptide of the present disclosure *(e.g.,* D-Arg-2',6'-Dmt-Lys-Phe-NH₂) may be administered to the subject. If a subject's C-reactive protein level is between about 1-3 mg/L, a medium level of an aromatic-cationic peptide of the present disclosure may be administered, and if a subject's level of C-reactive protein is between about 3-10 mg/L, a high level of an aromatic-cationic peptide of the present disclosure may be administered to the subject. A low dose of an aromatic-cationic peptide, such as D-Arg-2',6'-Dmt-Lys-Phe-NH₂, may result in a plasma concentration of less than about 50 ng/ml. A low dose of aromatic-cationic peptide may result in a plasma concentration of about 1, about 5, about 10, about 20, about 30, or about 40 ng/ml. A low dose or aromatic-cationic peptide may result in a plasma concentration of between about 10-40 ng/ml, between about 10-30 ng/ml, between about 10-20 ng/ml, between about 20-40 ng/ml, between about 20-30 ng/ml, between about 30-40 ng/ml, between about 30-50 ng/ml, or between about 40-50 ng/ml. A medium dose or an aromatic-cationic peptide, such as D-Arg-2',6'-Dmt-Lys-Phe-NH₂, may result in a plasma concentration of between about 50-500 ng/ml. A medium dose of aromatic-cationic peptide may result in a plasma concentration of about 50 ng/ml, about 100 ng/ml, about 150 ng/ml, about 200 ng/ml, about 250 ng/ml, about 300 ng/ml, about 350 ng/ml, about 400 ng/ml or about 450 ng/ml. A medium dose of aromatic-cationic peptide may result in a plasma concentration of between about 50-100 ng/ml, between about 50-200 ng/ml, between about 50-300 ng/ml, between about 50-400 ng/ml, between about 100-200 ng/ml, between about 100-300 ng/ml, between about 100-400 ng/ml, between about 100-500 ng/ml, between about 200-300 ng/ml, between about 200-400 ng/ml, between about 200-500 ng/ml, between about 300-400 ng/ml, between about 300-500 ng/ml or between about 400-500 ng/ml. A high dose of an aromatic-cationic peptide may result in a plasma concentration of between about 500-5000 ng/ml. A high dose of aromatic-cationic peptide may result in a plasma concentration of about 1000 ng/ml, about 1500 ng/ml, about 2000 ng/ml, about 2500 ng/ml, about 3000 ng/ml, about 3500 ng/ml, about 4000 ng/ml or about 4500 ng/ml. A high dose of aromatic-cationic peptide may result in a plasma concentration of between about 500-1000 ng/ml, between about 500-2000 ng/ml, between about 500-3000 ng/ml, between about 500-4000 ng/ml, between about 1000-2000 ng/ml, between about 1000-3000 ng/ml, between about 1000-4000 ng/ml, between about 1000-5000 ng/ml, between about 2000-3000 ng/ml, between about 2000-4000 ng/ml, between about 2000-5000 ng/ml, between about 3000-4000 ng/ml, between about 3000-5000 ng/ml or between about 4000-5000 ng/ml. A high dose of an aromatic-cationic peptide, such as D-Arg-2',6'-Dmt-Lys-Phe-NH₂, may result in a plasma concentration of greater than 5000 ng/ml. For example, a high dose of an aromatic-cationic peptide may result in a plasma concentration of about 6000 ng/ml, about 7000 ng/ml, about 8000 ng/ml, about 9000 ng/ml, or about 10,000 ng/ml. A high dose of an aromatic-cationic peptide may result in a plasma concentration of between about 5000-10,000 ng/ml, between about 5000-9000 ng/ml, between about 5000-8000 ng/ml, between about 5000-7000 ng/ml, between about 5000-6000 ng/ml, between about 6000-7000 ng/ml, between about 6000-8000 ng/ml, between about 6000-9000 ng/ml, between about 6000-10,000 ng/ml, between about 7000-8000 ng/ml, between about 7000-9000 ng/ml, between about 7000-10,000 ng/ml, between about 8000-9000 ng/ml, or between about 9000-10000 ng/ml. As described below, those skilled in the art will understand that dosage, toxicity and therapeutic efficacy of the aromatic-cationic peptides disclosed herein can be determined by standard pharmaceutical procedures in cell cultures or experimental animals.

For peptide dosage information, prophylactic or therapeutic treatment, CRP levels can be determined by any or a combination of methods known in the art. For example, , a hsCRP or cCRP an assay may be performed. A subject's CRP level may be between about 1-3 mg/L prior to peptide administration. The subject's CRP level may be between about 3-10 mg/L before peptide administration. The subject's CRP level may be greater than 10 mg/L before peptide administration. The subject's CRP level may beis about 2 fold greater than a control level before peptide administration. The subject's CRP level may be about 3 fold greater than a control level before peptide administration. The subject's CRP level may be about 4, 5 or 6 fold greater than a control before peptide administration.

### ROS, TNF-alpha, or IL-6 as biomarkers for peptide dosage.

TNF-α and IL-6 levels in plasma are typically reported in pg/ml and can range between about 0.1 pg/ml to about 2000 pg/ml depending on a pathophysiological condition one is working with. The total ROS amount in plasma is typically reported in RLU (relative light units/ml). The range of ROS level can be as low as about 1.0 RLU x 10⁵/ml and as high as about 200 RLU x 10⁵/ml depending on the physiological condition one is working with. Thus, ROS levels, TNF-alpha and/or IL-6 levels may be used to determine the dosage of aromatic-cationic peptide (*e.g*., D-Arg-2',6'-Dmt-Lys-Phe-NH₂) to administer to a subject. For example, if the level of ROS, TNF-alpha , or IL-6 protein in a subject is low (*e.g*., about 0.1-500 pg/ml for TNF-alpha, or IL-6 or about 1-70 RLU x 10⁵/ml for ROS), then a lower dose of aromatic-cationic peptide may be administered. Additionally or alternatively, if the level of ROS, TNF-alpha, or IL-6 protein in a subject is in a medium range (*e.g*., about 500-1000 pg/ml for TNF-alpha, or IL-6 or about 70-130 RLU x 10⁵/ml for ROS), then a medium dose of aromatic-cationic peptide may be administered. If the level of ROS, TNF-alpha, or IL-6 protein in a subject is high *(e.g.,* about 1000-2000 pg/ml for TNF-α or IL-6 or about 130-200 RLU x 10⁵/ml for ROS), then a high dose of aromatic-cationic peptide may be administered.

For peptide dosage information, prophylactic or therapeutic treatment, ROS levels can be determined by any or a combination of methods known in the art. For example, an absorbance, a fluorescence, or a luminescence assay may be performed. Asubject's ROS level may be between about 1 RLU x 10⁵/ml to about 5 RLU x 10⁵/ml prior to peptide administration. The subject's ROS level may be between about 5 RLU x 10⁵/ml to about 10 RLU x 10⁵/ml, or between about 10 RLU x 10⁵/ml to about 20 RLU x 10⁵/ml, or between about 20 RLU x 10⁵/ml to about 30 RLU x 10⁵/ml before peptide administration. The subject's ROS level may be greater than 30 RLU x 10⁵/ml before peptide administration. The subject's ROS level may be about 1.5 fold greater than a control level before peptide administration. The subject's ROS level may be about 2 fold greater than a control level before peptide administration. The subject's ROS level may be about 3, 4, 5, 6, 7, 8 fold or more greater than a control before peptide administration.

For peptide dosage information, prophylactic or therapeutic treatment, IL-6 levels can be determined by any or a combination of methods known in the art. For example, an ELISA or HPLC assays may be performed. A subject's IL-6 level may be about 8 pg/ml prior to peptide administration. The subject's IL-6 level may be between about 5-13 pg/ml before peptide administration. The subject's IL-6 level may be greater than 13 pg/ml before peptide administration. The subject's IL-6 level may be about 1.5 fold greater than a control level before peptide administration. The subject's IL-6 level may be about 2 fold greater than a control level before peptide administration. The subject's IL-6 level may be about 3, 4, 5, 6, 7, 8 fold or more greater than a control before peptide administration.

For peptide dosage information, prophylactic or therapeutic treatment, TNF-alpha levels can be determined by any or a combination of methods known in the art. For example, an ELISA or HPLC assays may be performed. A subject's TNF-alpha level may be about 1 pg/ml prior to peptide administration. The subject's TNF-alpha level may be between about 1.0-1.5 pg/ml before peptide administration. The subject's TNF-alpha level may be greater than 1.5 pg/ml before peptide administration. The subject's TNF-alpha level may be about 2 fold greater than a control level before peptide administration. The subject's TNF-alpha level may be about 3 fold greater than a control level before peptide administration. The subject's TNF-alpha level may be about 4, 5, 6, 7 or 8 fold or more greater than a control before peptide administration.

### Nt-pro BNP as a biomarker for peptide dosage

Nt-pro BNP in plasma can be reported in pg/ml and can range between about 1 pg/ml to about 3000 pg/ml depending on a pathophysiological condition one is working with. Thus, Nt-pro BNP may be used to determine the dosage of aromatic-cationic peptide *(e.g.,* D-Arg-2',6'-Dmt-Lys-Phe-NH₂) to administer to a subject. For example, if the level of Nt-pro BNP in a subject is low (*e.g*., about 1-1000 pg/ml), then a lower dose of aromatic-cationic peptide may be administered. Additionally or alternatively, if the level of Nt-pro BNP in a subject is in a medium range (*e.g.*, about 1000-2000 pg/ml), then a medium dose of aromatic-cationic peptide may be administered. Additionally or alternatively, if the level of Nt-pro BNP in a subject is high (*e.g.,* about 2000-3000 pg/ml), then a high dose of aromatic-cationic peptide may be administered.

For peptide dosage information, Nt-pro BNP levels can be determined by any or a combination of methods known in the art. For example, an ELISA or HPLC assays may be performed. A subject's Nt-pro BNP level may be about 250 pg/ml prior to peptide administration. The subject's Nt-pro BNP level may be between about 150-400 pg/ml before peptide administration. The subject's Nt-pro BNP level may be greater than 400 pg/ml before peptide administration. The subject's Nt-pro BNP level may be about 5 fold greater than a control level before peptide administration. The subject's Nt-pro BNP level may be about 6 fold greater than a control level before peptide administration. The subject's Nt-pro BNP level may be about 3, 4, 5, 6, 7, 8 fold or more greater than a control before peptide administration.

Nt-pro BNP levels may be measured as a biomarker for the decrease or prevention of LV remodeling after administration of aromatic-cationic peptide to a subject in need thereof. For example, administration with aromatic-cationic peptides disclosed herein, *e.g.,* D-Arg-2',6'-Dmt-Lys-Phe-NH₂, may reduce Nt-pro BNP by about 30%, about 40%, about 50% about 60%, about 70%, or about 80%. Administration with aromatic-cationic peptides may return Nt-pro BNP to or near baseline levels.

### Cardiac troponin I as a biomarker for peptide dosage

The subject's Nt-pro BNP level may be about 3, 4, 5, 6, 7, 8 fold or more greater than a control before peptide administration.

Cardiac troponin I levels may be measured as a biomarker for the decrease or prevention of LV remodeling after administration of aromatic-cationic peptide to a subject in need thereof. For example, administration with aromatic-cationic peptides disclosed herein, *e.g.*, D-Arg-2',6'-Dmt-Lys-Phe-NH₂, may reduce cardiac troponin I by about 10%, about 20%, about 30%, about 40%, about 50% about 60%, about 70%, about 80%, or about 90%. Administration with aromatic-cationic peptides may return cardiac troponin I to or near baseline levels.

### Modes of Administration and Effective Dosages

Any method known to those in the art for contacting a cell, organ or tissue with a peptide may be employed. Suitable methods include *in vitro, ex vivo, or in vivo* methods. *In vivo* methods typically include the administration of an aromatic-cationic peptide, such as those described above, to a mammal, suitably a human. When used *in vivo* for therapy, the aromatic-cationic peptides are administered to the subject in effective amounts *(i.e.,* amounts that have desired therapeutic effect). The dose and dosage regimen will depend upon the degree of the infection in the subject, the characteristics of the particular aromatic-cationic peptide used, *e.g.,* its therapeutic index, the subject, and the subject's history.

The effective amount may be determined during pre-clinical trials and clinical trials by methods familiar to physicians and clinicians. An effective amount of a peptide useful in the methods may be administered to a mammal in need thereof by any of a number of well-known methods for administering pharmaceutical compounds. The peptide may be administered systemically or locally.

The peptide may be formulated as a pharmaceutically acceptable salt. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (e.g., citric, gluconic, glycolic, lactic, lactobionic, malic, and tartaric acids), aliphatic monocarboxylic acids (*e.g.*, acetic, butyric, formic, propionic and trifluoroacetic acids), amino acids (*e.g*., aspartic and glutamic acids), aromatic carboxylic acids (*e.g.,* benzoic, p-chlorobenzoic, diphenylacetic, gentisic, hippuric, and triphenylacetic acids), aromatic hydroxyl acids (*e.g*., o-hydroxybenzoic, p-hydroxybenzoic, 1-hydroxynaphthalene-2-carboxylic and 3-hydroxynaphthalene-2-carboxylic acids), ascorbic, dicarboxylic acids (*e.g.*, fumaric, maleic, oxalic and succinic acids), glucuronic, mandelic, mucic, nicotinic, orotic, pamoic, pantothenic, sulfonic acids (*e.g.*, benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like. The salt may be an acetate or trifluoroacetate salt.

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (e.g., intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g*., vials of drug, vials of diluent, syringes and needles) for a treatment course *(e.g.,* 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g.*, gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser, which contains a suitable propellant, *e.g.,* a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. Transdermal administration may be performed my iontophoresis.

A therapeutic protein or peptide can be formulated in a carrier system. The carrier can be a colloidal system. The colloidal system can be a liposome, a phospholipid bilayer vehicle. The therapeutic peptide may be encapsulated in a liposome while maintaining peptide integrity. s one skilled in the art would appreciate, there are a variety of methods to prepare liposomes. *(See* Lichtenberg et al., Methods Biochem. Anal., 33:337-462 (1988); Anselem et al., Liposome Technology, CRC Press (1993)). Liposomal formulations can delay clearance and increase cellular uptake *(See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. The therapeutic peptide may be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. The polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. *(See* Reddy, *Ann. Pharmacother.,* 34(7-8):915-923 (2000)). A polymer formulation for human growth hormone (hGH) has been used in clinical trials. *(See* Kozarich and Rich, Chemical Biology, 2:548-552 (1998)).

Examples of polymer microsphere sustained release formulations are described in PCT publication WO 99/15154 (Tracy et al.), U.S. Pat. Nos. 5,674,534 and 5,716,644 (both to Zale et al.), PCT publication WO 96/40073 (Zale et al.), and PCT publication WO 00/38651 (Shah et al.). U.S. Pat. Nos. 5,674,534 and 5,716,644 and PCT publication WO 96/40073 describe a polymeric matrix containing particles of erythropoietin that are stabilized against aggregation with a salt.

The therapeutic compounds may be prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using known techniques. The materials can also be obtained commercially, *e.g.*, from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi et al., Cancer Lett., 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 *(i.e.,* the concentration of the test compound which achieves a half-maximal inhibition of symptoms) as determined in cell culture. Such information can be used to determine useful doses in humans accurately. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. A single dosage of peptide may range from 0.001-10,000 micrograms per kg body weight. Aromatic-cationic peptide concentrations in a carrier may range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

A therapeutically effective amount of an aromatic-cationic peptide may be defined as a concentration of peptide at the target tissue of 10⁻¹² to 10⁻⁶ molar, e.g., approximately 10⁻⁷ molar. This concentration may be delivered by systemic doses of 0.001 to 100 mg/kg or equivalent dose by body surface area. The schedule of doses would be optimized to maintain the therapeutic concentration at the target tissue, most preferably by single daily or weekly administration, but also including continuous administration (*e.g.*, parenteral infusion or transdermal application).

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. The mammal may be a human.

### Combination Therapy with an Aromatic-Cationic Peptide and Other Therapeutic Agents

The aromatic-cationic peptides may be combined with one or more additional agents for the prevention or treatment of heart failure. Drug treatment for heart failure typically involves diuretics, ACE inhibitors, digoxin (also called digitalis), calcium channel blockers, and beta-blockers. In mild cases, thiazide diuretics, such as hydrochlorothiazide at 25-50 mg/day or chlorothiazide at 250-500 mg/day, are useful. However, supplemental potassium chloride may be needed, since chronic diuresis causes hypokalemis alkalosis. Moreover, thiazide diuretics usually are not effective in patients with advanced symptoms of heart failure. Typical doses of ACE inhibitors include captopril at 25-50 mg/day and quinapril at 10 mg/day.

The aromatic-cationic peptide may be combined with an adrenergic beta-2 agonist. An "adrenergic beta-2 agonist" refers to adrenergic beta-2 agonists and analogues and derivatives thereof, including, for example, natural or synthetic functional variants, which have adrenergic beta-2 agonist biological activity, as well as fragments of an adrenergic beta-2 agonist having adrenergic beta-2 agonist biological activity. The term "adrenergic beta-2 agonist biological activity" refers to activity that mimics the effects of adrenaline and noradrenaline in a subject and which improves myocardial contractility in a patient having heart failure. Commonly known adrenergic beta-2 agonists include, but are not limited to, clenbuterol, albuterol, formeoterol, levalbuterol, metaproterenol, pirbuterol, salmeterol, and terbutaline.

The aromatic-cationic peptide may be combined with an adrenergic beta-1 antagonist. Adrenergic beta-1 antagonists and adrenergic beta-1 blockers refer to adrenergic beta-1 antagonists and analogues and derivatives thereof, including, for example, natural or synthetic functional variants which have adrenergic beta-1 antagonist biological activity, as well as fragments of an adrenergic beta-1 antagonist having adrenergic beta-1 antagonist biological activity. Adrenergic beta-1 antagonist biological activity refers to activity that blocks the effects of adrenaline on beta receptors. Commonly known adrenergic beta-1 antagonists include, but are not limited to, acebutolol, atenolol, betaxolol, bisoprolol, esmolol, and metoprolol.

Clenbuterol, for example, is available under numerous brand names including Spiropent^{®} (Boehinger Ingelheim), Broncodil^{®} (Von Boch I), Broncoterol^{®} (Quimedical PT), Cesbron^{®} (Fidelis PT), and Clenbuter^{®} (Biomedica Foscama). Similarly, methods of preparing adrenergic beta-1 antagonists such as metoprolol and their analogues and derivatives are well-known in the art. Metoprolol, in particular, is commercially available under the brand names Lopressor^{®} (metoprolol tartate) manufactured by Novartis Pharmaceuticals Corporation, One Health Plaza, East Hanover, N.J. 07936-1080. Generic versions of Lopressor^{®} are also available from Mylan Laboratories Inc., 1500 Corporate Drive, Suite 400, Canonsburg, Pa. 15317; and Watson Pharmaceuticals, Inc., 360 Mt. Kemble Ave. Morristown, N.J. 07962. Metoprolol is also commercially available under the brand name Toprol XL^{®}, manufactured by Astra Zeneca, LP.

An additional therapeutic agent may be administered to a subject in combination with an aromatic cationic peptide, such that a synergistic therapeutic effect is produced. Therefore, lower doses of one or both of the therapeutic agents may be used in treating heart failure, resulting in increased therapeutic efficacy and decreased side-effects.

In any case, the multiple therapeutic agents may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may vary from more than zero weeks to less than four weeks. In addition, the combination methods, compositions and formulations are not to be limited to the use of only two agents.

### EXAMPLES

The present invention is further illustrated by the following examples, which should not be construed as limiting in any way.

### Example 1 - Effects of Aromatic-Cationic Peptides on C-reactive protein in a Dog Model of Heart Failure

In this Example, the effect of the aromatic-cationic peptide D-Arg-2',6'-Dmt-Lys-Phe-NH₂ on reducing C-reactive protein levels in dogs with coronary microembolization-induced heart failure was investigated.

### Methods

Heart failure was induced in dogs via multiple sequential intracoronary microembolizations as described in Sabbah, et al., Am J Physiol. (1991) 260:H1379-84, herein incorporated by reference in its entirety.

Half the dogs were subsequently treated with the mitochondrial peptide; the other half were treated with drug vehicle and served as controls. Peptide treatment was started upon induction of heart failure (HF), defined as left ventricular ejection fraction of approximately 30%. Peripheral venous blood samples were obtained from all dogs at the following time points: 1) when the dogs were normal (baseline, prior to the induction of heart failure); 2) when the dogs were induced into heart failure; 3) at 6 weeks after initiating therapy with the mitochondrial peptide or vehicle (HF + peptide, or HF + vehicle) both administered once daily as subcutaneous injections; and 4) at 12 weeks after initiating therapy with the mitochondrial peptide or vehicle. The daily dose of the peptide was 0.5 mg/kg/day administered intravenously. Blood samples were drawn on EDTA anticoagulant and were centrifuged at 2,500 RPM and the plasma extracted, aliquoted in 1 ml volumes into crystat tubes and stored at -70°C until assayed. Once the follow-up was completed in all dogs, the plasma samples were thawed to room temperature and CRP was assayed using a canine specific ELISA kit (ALPCO: Cat# 41-CRPCA-E01). The calorimetric method was used to assess the amount of CRP in plasma. CRP concentration was expressed as µg/ml.

### Results

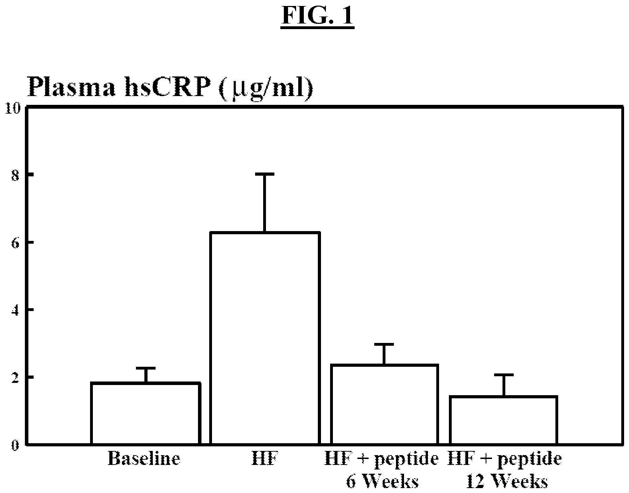

The results are shown in the Tables 10 and 11 below and in **FIG. 1****.** In the Tables, "BL" is baseline; "HF" is heart failure prior to initiating therapy; "6Wk" is 6 weeks after initiating therapy; "12 Wk" is 12 weeks after initiating therapy and marked the end of the study; and "SEM" is standard error of the mean. Values in the table are µg/ml CRP. As shown in the tables, in dogs treated with vehicle, plasma CRP increased in HF compared to baseline and tended to increase further at 6 week and 12 weeks after initiating subcutaneous injection with vehicle. In dogs treated with the mitochondrial peptide, CRP also increased when the dogs were induced into HF but treatment with the mitochondrial peptide reduced CRP at 6 weeks and reduced or normalized its concentration in plasma at 12 week.

| **Table 10: C-reactive protein concentration in control animals** | | | | |
|---|---|---|---|---|
| Dog # | **Vehicle (Control)** | | | |
| | **BL** | **Pre** | **6Wk** | **12 Wk** |
| 1 | 1.04 | 6.27 | 5.27 | 5.6 |
| 2 | 1.11 | 5.47 | 7.13 | 10.76 |
| 3 | 0.63 | 10.94 | 11.72 | 16.41 |
| **Mean** | **0.93** | **7.56** | **8.04** | **10.92** |
| **SEM** | **0.15** | **1.71** | **1.92** | **3.13** |

| **Table 11: C-reactive protein concentration in animals treated with peptide** | | | | |
|---|---|---|---|---|
| Dog # | **Mitochondrial peptide D-Arg-2',6'-Dmt-Lys-Phe-NH₂** | | | |
| | **BL** | **Pre** | **6Wk** | **12 Wk** |
| 1 | 1.02 | 5.07 | 1.12 | 0.51 |
| 2 | 2.54 | 9.71 | 3.23 | 2.69 |
| 3 | 1.89 | 4.06 | 2.69 | 1.07 |
| **Mean** | **1.82** | **6.28** | **2.35** | **1.42** |
| **SEM** | **0.44** | **1.74** | **0.63** | **0.65** |

As shown in **FIG. 1****,** plasma CRP levels as determined using a high-sensitivity assay increased about 3 fold in the heart failure subjects. Treatment with D-Arg-2',6'-Dmt-Lys-Phe-NH₂ for six weeks reduced CRP levels, while treatment for 12 weeks reduced CRP levels in the treated heart failure subjects to normal or near normal levels.

The results show that D-Arg-2',6'-Dmt-Lys-Phe-NH₂ is useful for lowering CRP levels in heart failure subjects, thereby reducing the risk of a future heart failure event or recurrence, reducing the severity of future heart failure, and/or preventing heart failure in an undiagnosed subjects. As such, the aromatic-cationic peptide D-Arg-2',6'-Dmt-Lys-Phe-NH₂ is useful for reducing CRP levels, thereby preventing or treating heart failure in mammalian subjects.

### Example 2 -Treatment or Prevention of Heart Failure in Human

Forty human subjects diagnosed with heart failure and having elevated CRP levels in the range of 3-10 mg/L, as determined by either a hsCRP or cCRP assay, will be randomly divided into four groups and will be treated with vehicle (control subject group), or aromatic-cationic peptide D-Arg-2',6'-Dmt-Lys-Phe-NH₂ at a dosage of 1 mg/kg/d, 3 mg/kg/d or 5 mg/kg/d. Peptide or vehicle will be administered subcutaneously in three separate doses per day, every 8 hours. CRP levels will be evaluated during the course of 12-week treatment.

### Results

Treatment with D-Arg-2',6'-Dmt-Lys-Phe-NH₂ is predicted to reduce CRP levels in treated subjects. It is anticipated that treatment with D-Arg-2',6'-Dmt-Lys-Phe-NH₂ will reduce CRP levels to normal range *(e.g.,* less than 1 mg/L), and/or will reduce the level of CRP from a high risk level *(e.g.,* 3-10 mg/L) to an intermediate risk level *(e.g.,* 1-3 mg/L), thereby reducing the risk of a future heart failure event or recurrence, reducing the severity of heart failure, and/or preventing heart failure in an undiagnosed subjects or subjects at risk of heart failure. As such, results are anticipated to show that aromatic-cationic peptide D-Arg-2',6'-Dmt-Lys-Phe-NH₂ is useful in methods for lowering CRP levels in humans, and for preventing or treating heart failure in human subjects.

### Example 3 - Effects of Aromatic-Cationic Peptides on ROS in a Dog Model of Heart Failure

In this example, the effect of the aromatic-cationic peptide D-Arg-2',6'-Dmt-Lys-Phe-NH₂ on ROS levels in dogs with coronary microembolization-induced heart failure was investigated.

### Methods

Heart failure was induced as described in Example 1. Ten dogs were used in the experiment to determine the effect of D-Arg-2',6'-Dmt-Lys-Phe-NH₂ on ROS levels. Half the dogs were treated with the peptide; the other half were treated with drug vehicle and served as controls. Peptide treatment was started upon induction of heart failure (HF), defined as left ventricular ejection fraction of approximately 30%. The daily dose of the peptide was 0.5 mg/kg/day administered intravenously. Blood samples were drawn on EDTA anticoagulant and were centrifuged at 2,500 RPM and the plasma extracted, aliquoted in 1 ml volumes into crystat tubes and stored at -70°C until assayed. Once the follow-up was completed in all dogs, the plasma samples were thawed to room temperature and ROS was assayed. To assess ROS levels in the plasma, luminol (5-amino-2, 3 dihydro-1, 4 phtalazindione) was added to the samples. ROS levels were then assessed by chemiluminescence activity.

### Results

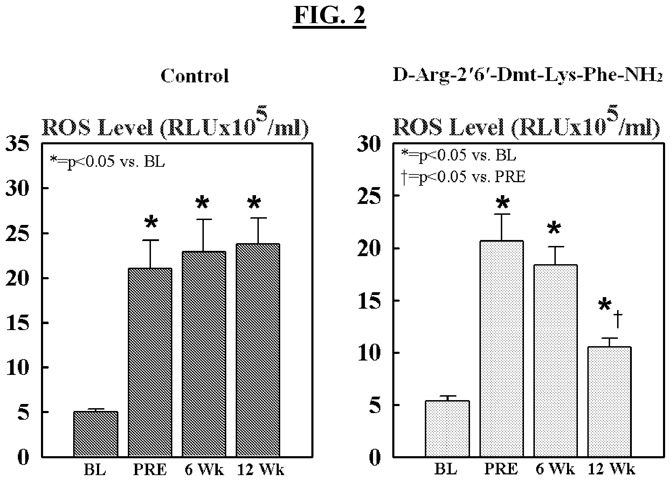

The results are shown in the Tables 12 and 13 below and in **FIG. 2****.** In the tables, "BL" is baseline; "PRE" is heart failure prior to initiating therapy; "6Wk" is 6 weeks after initiating therapy; "12 Wk" is 12 weeks after initiating therapy and marked the end of the study; and "SEM" is standard error of the mean. "ND" indicates that no plasma was assayed. Values in the table are RLUx10⁵/ml ROS, wherein RLU stands for relative light units. As shown in the tables, in dogs treated with vehicle, plasma ROS increased in HF compared to baseline and tended to increase further at 6 weeks and 12 weeks after initiating subcutaneous injection with vehicle. In dogs treated with the mitochondrial peptide, ROS also increased when the dogs were induced into HF but treatment with the mitochondrial peptide reduced ROS at 6 weeks and reduced or normalized its concentration in plasma at 12 week.

| **Table 12: Reactive oxygen species concentration in control animals** | | | | |
|---|---|---|---|---|
| Dog # | **Vehicle (Control)** | | | |
| | **BL** | **PRE** | **6Wk** | **12 Wk** |
| 1 | 4.6 | ND | ND | 20.1 |
| 2 | 5.5 | 14.4 | 14.7 | 16.8 |
| 3 | 3.8 | 22.5 | 26.0 | 27.3 |
| 4 | 5.4 | 18.4 | 19.5 | 21.5 |
| 5 | 5.6 | 29.0 | 31.3 | 33.2 |
| **Mean** | **5.0** | **21.1** | **22.9** | **23.8** |
| **SEM** | **0.3** | **3.1** | **3.6** | **2.9** |

| **Table 13: Reactive oxygen species concentration in animals treated with peptide** | | | | |
|---|---|---|---|---|
| Dog # | **Mitochondrial peptide D-Arg-2',6'-Dmt-Lys-Phe-NH₂** | | | |
| | **BL** | **PRE** | **6Wk** | **12 Wk** |
| 1 | 6.8 | ND | ND | 9.0 |
| 2 | 5.7 | 14.6 | 15.1 | 12.4 |
| 3 | 4.7 | 26.0 | 23.3 | 10.5 |
| 4 | 5.5 | 23.6 | 18.4 | 12.4 |
| 5 | 3.9 | 18.5 | 16.6 | 8.3 |
| **Mean** | **5.3** | **20.7** | **18.4** | **10.5** |
| **SEM** | **0.5** | **2.6** | **1.8** | **0.8** |

As shown in **FIG. 2****,** plasma ROS levels increased over 4 fold in the heart failure subjects. Treatment with D-Arg-2',6'-Dmt-Lys-Phe-NH₂ for six weeks reduced ROS levels, while treatment for 12 weeks reduced ROS levels in the treated heart failure subjects to near normal levels.

The results show that D-Arg-2',6'-Dmt-Lys-Phe-NH₂ is useful for lowering ROS levels in heart failure subjects, thereby reducing the risk of a future heart failure event or recurrence, reducing the severity of future heart failure, and/or preventing heart failure in an undiagnosed subjects. As such, the aromatic-cationic peptide D-Arg-2',6'-Dmt-Lys-Phe-NH₂ is useful for reducing ROS levels, and preventing or treating heart failure in mammalian subj ects.

### Example 4 - Effects of Aromatic-Cationic Peptides on IL-6 in a Dog Model of Heart Failure

In this example, the effect the aromatic-cationic peptide D-Arg-2',6'-Dmt-Lys-Phe-NH₂ on interleukin-6 (IL-6) levels in dogs with coronary microembolization-induced heart failure was investigated.

### Methods

The methods of Example 1 were used to induce heart failure, administer peptide or vehicle and collect plasma. Fourteen dogs were used in this example. Half the dogs were treated with the mitochondrial peptide; the other half were treated with drug vehicle and served as controls. Peptide treatment was started in both groups upon induction of heart failure (HF), defined as left ventricular ejection fraction of approximately 30%.

IL-6 was quantified in EDTA-plasma using the double antibody sandwich ELISA technique. Briefly, IL-6 (Canine IL-6 Duo Set, Cat#DY1609) was purchased from R&D Systems. The assay was performed based on instructions provided by the supplier with minor modifications. The amount of EDTA-plasma incubated with the antibody plate was 150 µl and incubation time was 18 hours at 4°C. Using standard curves and software, the concentration of IL-6 was expressed as pg/ml.

### Results

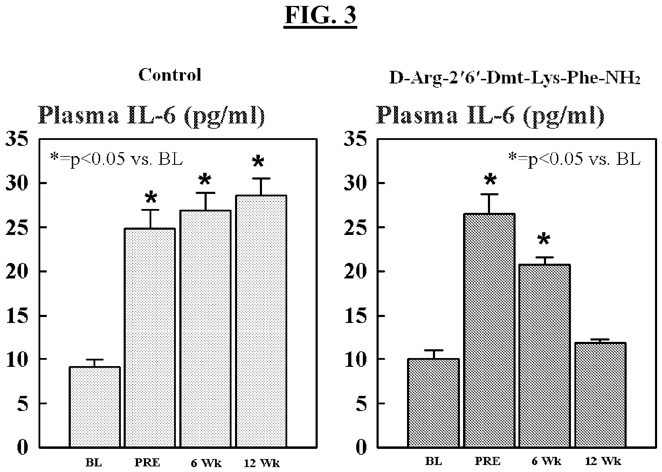

The results are shown in the Tables 14 and 15 below and in **FIG. 3****.** In the tables, "BL" is baseline; "PRE" is heart failure prior to initiating therapy; "6Wk" is 6 weeks after initiating therapy; "12 Wk" is 12 weeks after initiating therapy and marked the end of the study; and "SEM" is standard error of the mean. Values in the table are pg/ml IL-6. As shown in the tables, in dogs treated with vehicle, plasma IL-6 increased in HF compared to baseline and tended to increase further at 6 week and 12 weeks after initiating subcutaneous injection with vehicle. In dogs treated with the mitochondrial peptide, IL-6 also increased when the dogs were induced into HF but treatment with the mitochondrial peptide reduced IL-6 at 6 weeks and reduced or normalized its concentration in plasma at 12 week.

| **Table 14: IL-6 concentration in control animals** | | | | |
|---|---|---|---|---|
| Dog # | **Vehicle (Control)** | | | |
| | **BL** | **PRE** | **6Wk** | **12 Wk** |
| 1 | 10.3 | 28.4 | 30.1 | 31.0 |
| 2 | 9.0 | 17.6 | 22.1 | 24.7 |
| 3 | 11.6 | 30.6 | 34.3 | 34.3 |
| 4 | 7.0 | 18.5 | 20.3 | 21.4 |
| 5 | 8.2 | 21.3 | 22.6 | 24.7 |
| 6 | 11.8 | 28.5 | 29.2 | 30.2 |
| 7 | 5.8 | 29.2 | 29.8 | 33.8 |
| **Mean** | **9.1** | **24.9** | **26.9** | **28.6** |
| **SEM** | **0.9** | **2.1** | **2.0** | **1.9** |

| **Table 15: IL-6 concentration in animals treated with peptide** | | | | |
|---|---|---|---|---|
| Dog # | **Mitochondrial peptide D-Arg-2',6'-Dmt-Lys-Phe-NH₂** | | | |
| | **BL** | **PRE** | **6Wk** | **12 Wk** |
| 1 | 10.3 | 31.2 | 19.6 | 12.5 |
| 2 | 12.5 | 29.4 | 20.5 | 11.9 |
| 3 | 13.0 | 30.4 | 19.6 | 11.4 |
| 4 | 9.1 | 29.3 | 18.8 | 11.7 |
| 5 | 5.8 | 18.5 | 23.4 | 11.3 |
| 6 | 7.0 | 29.2 | 24.3 | 13.6 |
| 7 | 12.6 | 18.0 | 19.5 | 11.5 |
| **Mean** | **10.1** | **26.6** | **20.8** | **12.0** |
| **SEM** | **1.1** | **2.2** | **0.8** | **0.3** |

As shown in **FIG. 3****,** plasma IL-6 levels, as determined using a high-sensitivity assay, increased about 3 fold in the heart failure subjects. Treatment with D-Arg-2',6'-Dmt-Lys-Phe-NH₂ for six weeks reduced IL-6 levels, while treatment for 12 weeks reduced IL-6 levels in the treated heart failure subjects to near normal levels.

The results show that D-Arg-2',6'-Dmt-Lys-Phe-NH₂ is useful for lowering IL-6 levels in heart failure subjects, thereby reducing the risk of a future heart failure event or recurrence, reducing the severity of future heart failure, and/or preventing heart failure in an undiagnosed subjects. As such, the aromatic-cationic peptide D-Arg-2',6'-Dmt-Lys-Phe-NH₂ is useful for reducing IL-6 levels, and preventing or treating heart failure in mammalian subj ects.

### Example 5 - Effects of Aromatic-Cationic Peptides on TNF-alpha in a Dog Model of Heart Failure

In this example, the effect of the aromatic-cationic peptide D-Arg-2',6'-Dmt-Lys-Phe-NH₂ on tumor necrosis factor alpha levels in dogs with coronary microembolization-induced heart failure was investigated.

### Methods

The methods of Example 1 was used induce heart failure, administer peptide or vehicle, and to collect plasma samples for analysis. Fourteen dogs were used in this example. Half the dogs were treated with the peptide; the other half were treated with drug vehicle and served as controls. Peptide treatment was started in both groups upon induction of heart failure (HF), defined as left ventricular ejection fraction of approximately 30%.

TNF-alpha was quantified in EDTA-plasma using a double antibody sandwich ELISA. Briefly, TNF-alpha (Canine TNFα Duo Sett, Cat# DY1507) was purchased from R&D Systems. The assay was performed based on instructions provided by the supplier with minor modifications. The amount of EDTA-plasma incubated with antibody plate was 150 µl and incubation time was 18 hours at 4°C. Using standard curves and software, the concentration of TNFα was expressed as pg/ml.

### Results

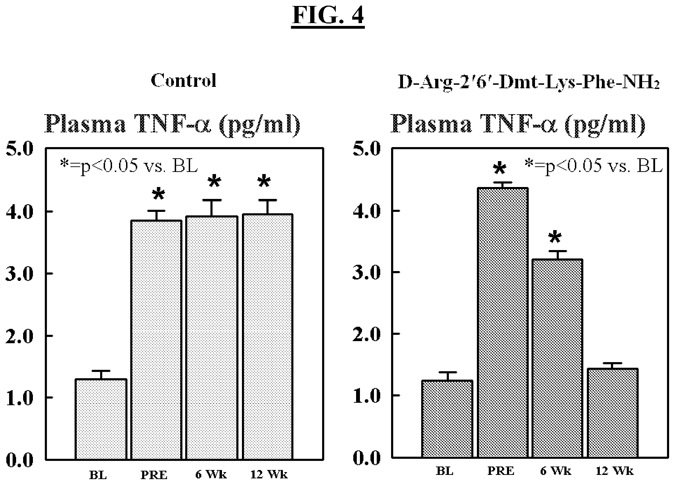

The results are shown in the Tables 16 and 17 below and in **FIG. 4****.** In the tables, "BL" is baseline; "PRE" is heart failure prior to initiating therapy; "6Wk" is 6 weeks after initiating therapy; "12 Wk" is 12 weeks after initiating therapy and marked the end of the study; and "SEM" is standard error of the mean. Values in the table are pg/ml TNF-alpha. As shown in the tables, in dogs treated with vehicle, plasma TNF-alpha increased in HF compared to baseline and tended to increase further at 6 week and 12 weeks after initiating subcutaneous injection with vehicle. In dogs treated with the peptide, TNF-alpha also increased when the dogs were induced into HF but treatment with the mitochondrial peptide reduced TNF-alpha at 6 weeks and reduced or normalized its concentration in plasma at 12 week.

| **Table 16: TNF-α concentration in control animals** | | | | |
|---|---|---|---|---|
| Dog # | **Vehicle (Control)** | | | |
| | **BL** | **PRE** | **6Wk** | **12 Wk** |
| 1 | 0.99 | 3.78 | 4.37 | 4.80 |
| 2 | 0.84 | 3.86 | 4.55 | 3.93 |
| 3 | 1.72 | 3.68 | 3.95 | 3.68 |
| 4 | 1.15 | 3.47 | 3.86 | 2.94 |
| 5 | 1.85 | 3.54 | 2.53 | 3.76 |
| 6 | 1.30 | 4.74 | 3.73 | 4.00 |
| 7 | 1.17 | 3.91 | 4.48 | 4.57 |
| **Mean** | **1.29** | **3.85** | **3.92** | **3.95** |
| **SEM** | **0.14** | **0.16** | **0.26** | **0.23** |

| **Table 17: TNF-α concentration in animals treated with peptide** | | | | |
|---|---|---|---|---|
| Dog # | **Mitochondrial peptide D-Arg-2',6'-Dmt-Lys-Phe-NH₂** | | | |
| | **BL** | **PRE** | **6Wk** | **12 Wk** |
| 1 | 1.30 | 4.59 | 3.72 | 1.69 |
| 2 | 1.11 | 4.31 | 3.12 | 1.53 |
| 3 | 1.54 | 4.42 | 2.60 | 1.35 |
| 4 | 0.90 | 4.26 | 3.27 | 1.14 |
| 5 | 0.97 | 3.96 | 3.08 | 1.42 |
| 6 | 1.02 | 4.26 | 3.50 | 1.18 |
| 7 | 1.85 | 4.69 | 3.16 | 1.75 |
| **Mean** | **1.24** | **4.36** | **3.21** | **1.44** |
| **SEM** | **0.13** | **0.09** | **0.13** | **0.09** |

As shown in **FIG. 4****,** plasma TNF-alpha levels increased about 4 fold in the heart failure subjects. Treatment with D-Arg-2',6'-Dmt-Lys-Phe-NH₂ for six weeks reduced TNF-alpha levels, while treatment for 12 weeks reduced TNF-alpha levels in the treated heart failure subjects to near normal levels.

The results show that D-Arg-2',6'-Dmt-Lys-Phe-NH₂ is useful for lowering TNF-alpha levels in heart failure subjects, thereby reducing the risk of a future heart failure event or recurrence, reducing the severity of future heart failure, and/or preventing heart failure in an undiagnosed subjects. As such, the aromatic-cationic peptide D-Arg-2',6'-Dmt-Lys-Phe-NH₂ is useful for reducing TNF-alpha levels, and preventing or treating heart failure in mammalian subjects.

### Example 6 - D-Arg-2',6'-Dmt-Lys-Phe-NH₂ administered post-myocardial infarction improves LV function

This study will demonstrate that chronic therapy with D-Arg-2',6'-Dmt-Lys-Phe-NH₂, begun at 2 hours post induction of heart failure by a transmural, non reperfused infarct in the rat, can improve outcome. Since D-Arg-2',6'-Dmt-Lys-Phe-NH₂ treatment will start at two hours after permanent coronary occlusion, any benefit will be independent of phenomena such as no-reflow reduction. Two hours after coronary occlusion, all or nearly all cells destined to die due to ischemic necrosis have died in the rat model. This study measures the ability of D-Arg-2',6'-Dmt-Lys-Phe-NH₂ to reduce LV volumes, improve fractional shortening and ejection fraction, reduce infarct expansion, improve survival, improve hemodynamics, and reduce lung volumes.

### Methods

Rats are anesthetized, ventilated, and a thoracotomy performed in the left 4^{th} intercostal space. Temperature is maintained at 36°C by placing the rats on a heating pad during the procedure. The pericardium is excised and the proximal left coronary artery is isolated and permanently occluded with a suture. Coronary artery occlusion is confirmed by cyanosis and akinesis of the anterior wall of the ventricle. The chest is closed, air evacuated, and the rats are allowed to recover. Analgesia is administered per the veterinarian. An echocardiogram is obtained at approximately 15 minutes post coronary artery occlusion. At 2 hours rats are randomized to receive chronic daily D-Arg-2',6'-Dmt-Lys-Phe-NH₂ (delivered subcutaneously by an Alzet Osmotic Pump - 3 mg/kg/day) or water. The Osmotic Pump delivers approximately 0.15 µl/hr for 6 weeks (model 2006; 200 µl). The Alzat pump is implanted subcutaneously between the shoulder blades while the rat is still anesthetized. After 6 weeks the rats are re-anesthetized, weighed, and a second echocardiogram is obtained under anesthesia. Cut downs are performed to isolate the carotid artery and jugular vein. Heart rate and blood pressure are measured. A Millar catheter is inserted into the left ventricle and LV systolic pressure, LV end diastolic pressure, +dP/dt, and -dP/dt are measured. A left ventriculogram is performed using IV fluoroscopic contrast in order to determine LV stroke volume and ejection fraction. Under deep anesthesia, the heart is excised, weighed, and pressure fixed at 11 mmHg with formalin. The lungs are also excised and weighed. Postmortem LV volume is measured by filling the LV cavity with fluid and measuring the total fluid. The hearts are sliced into four transverse sections and histologic slides are prepared and stained with hematoxylin and eosin and with picrosirius red, which stains collagen. Quantitative histologic analysis includes: total circumference, scar circumference, non-infarcted wall circumference, total LV area, total LV cavity area, LV wall thickness (at several points), non-infarcted wall thickness; myocardial infarct expansion index.

### Results

### LV fractional shortening by echocardiography

It is anticipated that the left ventricular fractional shortening (LVFS) will improve in the D-Arg-2',6'-Dmt-Lys-Phe-NH₂ treated group, as compared to the untreated group.

### LV stroke volume and ejection fraction by LV ventriculography

It is anticipated that there will be a higher LV stroke volume and LV ejection fraction in the D-Arg-2',6'-Dmt-Lys-Phe-NH₂ treated group, as compared to the untreated group.

### Hemodynamics

No significant differences are anticipated in heart rate, systolic and diastolic blood pressure between the two groups at 6 weeks after treatment.

### Post-mortem LV volumes

It is anticipated that there will be a lower post-mortem LV volume in the D-Arg-2',6'-Dmt-Lys-Phe-NH₂ -treated group as compared to the control group.

### Scar circumference, scar thickness, and expansion index

It is anticipated that the LV non-scar circumference will be longer in the D-Arg-2',6'-Dmt-Lys-Phe-NH₂ treated group as compared to the water group. Additionally, the scar circumference is anticipated to be smaller in the D-Arg-2',6'-Dmt-Lys-Phe-NH₂ treated group as compared to the water group. It is anticipated that the scar circumference, expressed as percentage of total LV circumference, will be smaller in the D-Arg-2',6'-Dmt-Lys-Phe-NH₂ group as compared to the water. The scar thickness, septum thickness and expansion index expressed as: [LV cavity area/Total LV area x Septum thickness/Scar thickness], is anticipated to be comparable between the two groups.

### Lung weights (a measure of fluid overload)

The lung dry and wet weight is measured, and the ratio of dry/wet is anticipated to be similar in between the two groups.

These results will show that aromatic-cationic peptides of the present technology, such as D-Arg-2',6'-Dmt-Lys-Phe-NH₂, or a pharmaceutically acceptable salt thereof, such as acetate or trifluoroacetate salt, are useful in the prevention of LV remodeling and improvement of LV function. In particular, these results will show that aromatic-cationic peptides of the present invention, such as D-Arg-2',6'-Dmt-Lys-Phe-NH₂, or a pharmaceutically acceptable salt thereof, such as acetate or trifluoroacetate salt, are useful in methods comprising administration of the peptide to subjects in need of decreased left ventricular remodeling and improved LV function.

### Example 7 - Effects of D-Arg-2',6'-Dmt-Lys-Phe-NH₂ on post-infarction remodeling and cardiac function in a rodent model of heart failure

In this study, D-Arg-2',6'-Dmt-Lys-Phe-NH₂ is tested to see if it will improve cardiac function and result in beneficial mitochondrial gene expression in a post-infarct model of heart failure.

### Methods

Rats will undergo the permanent coronary artery ligation, as described in Example 6. The rats will be split into two groups and treated for six weeks with either 200-300 ng/ml of D-Arg-2',6'-Dmt-Lys-Phe-NH₂ or 0.9% NaCl (saline) continuously through mini-osmotic pumps, which are implanted into each animal.

After the six week period, LV function is assessed with echocardiography. Additionally, the hearts are excised and the heart tissue is analyzed for LV chamber volume using tetrazolium salt staining. Heart tissue in the border zone and remote areas around the infarct are also harvested and undergo gene array analysis to determine the expression levels of genes involved in mitochondrial metabolism.

### Results

It is anticipated that chronic treatment with D-Arg-2',6'-Dmt-Lys-Phe-NH₂ will reduce LV dilation in a post-infarction model of heart failure.

These results will show that aromatic-cationic peptides, such as D-Arg-2',6'-Dmt-Lys-Phe-NH₂, or a pharmaceutically acceptable salt thereof, such as acetate or trifluoroacetate salt are useful in the treatment of LV remodeling and heart failure. Accordingly, the peptides are useful in methods comprising administering aromatic-cationic peptides to a subject suffering from heart failure or post myocardial infarct.

### Example 8 - Effects of Aromatic-Cationic Peptides on NT-pro BNP levels in a Dog Model of Heart Failure

In this Example, the effect of the aromatic-cationic peptide D-Arg-2',6'-Dmt-Lys-Phe-NH₂ on reducing NT-pro BNP levels in dogs with coronary microembolization-induced heart failure was investigated.

### Methods

Heart failure was induced in dogs via multiple sequential intracoronary microembolizations as described in Sabbah, et al., Am J Physiol. (1991) 260:H1379-84, herein incorporated by reference in its entirety and summarized in Example 1.

Heart failure was induced as described in Example 1. Ten dogs were used in the experiment to determine the effect of D-Arg-2',6'-Dmt-Lys-Phe-NH₂ on NT-pro BNP levels. Half the dogs were treated with the peptide; the other half were treated with drug vehicle and served as controls. Peptide treatment was started upon induction of heart failure (HF), defined as left ventricular ejection fraction of approximately 30%. The daily dose of the peptide was 0.5 mg/kg/day administered intravenously. Blood samples were drawn on EDTA anticoagulant and were centrifuged at 2,500 RPM and the plasma extracted, aliquoted in 1 ml volumes into crystat tubes and stored at -70°C until assayed. Once the follow-up was completed in all dogs, the plasma samples were thawed to room temperature and NT-pro BNP was assayed.

Nt-pro BNP (pg/ml) was determined in EDTA-plasma on the principle of the double antibody sandwich Enzyme-linked immunosorbent assay (ELISA). The assay was performed based upon the instructions came along with the assay kit (NT-pro BNP, Kamiya Biomedical Company, Cat# KT-23770). Using standard curves with the help of Software (MasterPlex-2010), the concentration of each biomarker was determined.

### Results

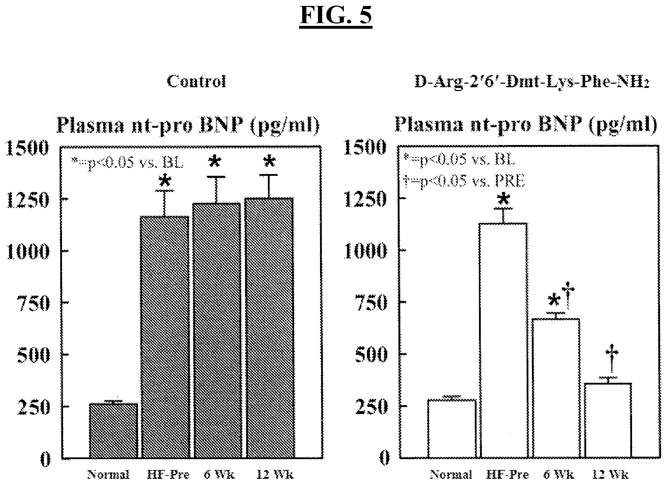

The results are shown in the Tables 18 and 19 below and in **FIG. 5****.** In the tables, normal is baseline before heart failure; "HF-Pre" is heart failure prior to initiating therapy; "6 Wk" is 6 weeks after initiating therapy; "12 Wk" is 12 weeks after initiating therapy and marked the end of the study; "AVG" is the average; "SD" is standard deviation; "SEM" is standard error of the mean; and "NS is not significant. Values in the table are pg/ml NT-pro BNP. As shown in the tables, in dogs treated with vehicle, plasma NT-pro BNP, on average, increased in HF compared to baseline and tended to increase further at 6 week and 12 weeks after initiating subcutaneous injection with vehicle. In dogs treated with the mitochondrial peptide, NT-pro BNP also increased when the dogs were induced into HF but treatment with the mitochondrial peptide reduced NT-pro BNP at 6 weeks and reduced or normalized its concentration in plasma at 12 week.

| **Table 18: Nt-pro BNP levels (pg/ml) in Plasma of CHF Dogs in control animals** | | | | |
|---|---|---|---|---|
| Dog # | **Vehicle (Control)** | | | |
| | **Normal** | **HF-Pre** | **6Wk** | **12 Wk** |
| 1 | 324 | 1814 | 1492 | 1363 |
| 2 | 224 | 1418 | 1588 | 1568 |
| 3 | 211 | 997 | 1363 | 1372 |
| 4 | 267 | 1060 | 1464 | 1588 |
| 5 | 244 | 950 | 871 | 930 |
| 6 | 294 | 991 | 1104 | 1082 |
| 7 | 271 | 911 | 712 | 850 |
| **AVG** | **262** | **1163** | **1228** | **1250** |
| **SD** | **39** | **333** | **337** | **298** |
| **SEM** | **15** | **126** | **127** | **113** |
| **P-value** vs. **Normal** | | **<.05** | **<.05** | **<.05** |
| **P-value vs. HF-Pre** | | | **NS** | **NS** |

| **Table 19: C-reactive protein concentration in animals treated with peptide** | | | | |
|---|---|---|---|---|
| Dog # | **Mitochondrial peptide D-Arg-2',6'-Dmt-Lys-Phe-NH₂** | | | |
| | **BL** | **Pre** | **6Wk** | **12 Wk** |
| 1 | 289 | 1018 | 695 | 417 |
| 2 | 265 | 1090 | 593 | 393 |
| 3 | 346 | 941 | 654 | 423 |
| 4 | 257 | 1512 | 695 | 299 |
| 5 | 203 | 1223 | 559 | 422 |
| 6 | 271 | 1078 | 796 | 234 |
| 7 | 320 | 1034 | 680 | 294 |
| **AVG** | **278** | **1128** | **667** | **354** |
| **SD** | **46** | **190** | **77** | **77** |
| **SEM** | **17** | **72** | **29** | **29** |
| **P-value vs. Normal** | | **<.05** | **<.05** | **NS** |
| **P-value vs. HF-Pre** | | | **<.05** | **<.05** |

As shown in **FIG. 5****,** plasma NT-pro BNP levels as determined using double antibody ELISA increased about 4 fold in the heart failure subjects. Treatment with D-Arg-2'6'-Dmt-Lys-Phe-NH₂ for six weeks reduced NT-pro BNP levels, while treatment for 12 weeks reduced NT-pro BNP levels in the treated heart failure subjects to normal or near normal levels.

The results show that D-Arg-2',6'-Dmt-Lys-Phe-NH₂ lowers NT-pro BNP levels in heart failure subjects. The decrease in NT-pro BNP correlates to a decrease in BNP, since both are release in equimolar concentration after the cleavage of proBNP, thereby indicating a decrease in the stretching of cardiomyocytes. As such, the aromatic-cationic peptide D-Arg-2',6'-Dmt-Lys-Phe-NH₂ is useful for preventing or treating LV remodeling in mammalian subjects.

### Example 9 - Effects of Aromatic-Cationic Peptides on Mitochondria ATP-sensitive potassium Channel (mK ATP) in a Dog Model of Heart Failure

In this Example, the effect of the aromatic-cationic peptide D-Arg-2',6'-Dmt-Lys-Phe-NH₂ on increasing mK ATP activity in dogs with coronary microembolization-induced heart failure was investigated.

### Methods

Heart failure was induced in dogs via multiple sequential intracoronary microembolizations as described in Sabbah, et al., Am J Physiol. (1991) 260:H1379-84, herein incorporated by reference in its entirety.

Heart failure was induced as described in Example 1. Ten dogs were used in the experiment to determine the effect of D-Arg-2',6'-Dmt-Lys-Phe-NH₂ on mk ATP activity. Half the dogs were treated with the peptide (n=5); the other half were treated with drug vehicle and served as controls (n=5). Peptide treatment was started upon induction of heart failure (HF), defined as left ventricular ejection fraction of approximately 30%. The daily dose of the peptide was 0.5 mg/kg/day administered intravenously for three months. Subcutaneous daily injections of saline were administered to the controls. Left ventricular tissue was harvested at the end of the three months of treatment. Mitochondria were isolated from the tissue. MK ATP activation was measured using the thallium-sensitive fluorophore assay kit and quantified in relative fluorescence units (RFU) per mg protein. Mitochondrial ATP to ADP ratio was measured using the bioluminescent ApoSENSOR^{™} assay kit (Enzo Life Sciences, Farmingdale, NY).

### Results

Treatment with D-Arg-2',6'-Dmt-Lys-Phe-NH₂ resulted in a significant increase in the ATP/ADP (0.38 ± 0.04 vs. 1.16 ± 0.15, p<0.05). Treatment with D-Arg-2',6'-Dmt-Lys-Phe-NH₂ also resulted in a significant increase in activation of mK ATP (1372 ± 112 vs. 2775 ± 254, p<0.05).

The results show that D-Arg-2',6'-Dmt-Lys-Phe-NH₂ increases mK ATP activity in heart failure subjects, thereby reducing the risk of a future heart failure event or recurrence, reducing the severity of future heart failure, and/or preventing heart failure in an undiagnosed subjects. As such, the aromatic-cationic peptide D-Arg-2',6'-Dmt-Lys-Phe-NH₂ is useful for increasing mK ATP activity, and preventing or treating heart failure in mammalian subjects.

### Example 10 - Effects of Aromatic-Cationic Peptides on cardiac troponin I levels in a Dog Model of Heart Failure

In this example, the effect of the aromatic-cationic peptide D-Arg-2',6'-Dmt-Lys-Phe-NH₂ on cardiac troponin I levels in dogs with coronary microembolization-induced heart failure is investigated.

### Methods

Heart failure is induced as described in Example 1. Ten dogs are used in the experiment to determine the effect of D-Arg-2',6'-Dmt-Lys-Phe-NH₂ on cardiac troponin I levels. Half the dogs are treated with the peptide; the other half are treated with drug vehicle and served as controls. Peptide treatment is started upon induction of heart failure (HF), defined as left ventricular ejection fraction of approximately 30%. The daily dose of the peptide was 0.5 mg/kg/day is administered intravenously. Blood samples are drawn on EDTA anticoagulant and are centrifuged at 2,500 RPM and the plasma extracted, aliquoted in 1 ml volumes into crystat tubes and stored at -70°C until assayed. Cardiac troponin I levels are measured for a baseline, at six weeks after treatment, and at twelve weeks after treatment. Once the assay is completed in all dogs, the plasma samples are thawed to room temperature and cardiac troponin I is assayed. Baseline, six weeks treatment, and twelve weeks treatment are compared to normal controls.

### Results

It is anticipated that treatment with D-Arg-2',6'-Dmt-Lys-Phe-NH₂ will reduce cardiac troponin I levels after six weeks of treatment as compared to untreated and that after 12 weeks of treatment it is anticipated that cardiac troponin I levels will be near normal levels.

The results will shows that D-Arg-2',6'-Dmt-Lys-Phe-NH₂ is useful for lowering cardiac troponin I levels in heart failure subjects, thereby reducing the risk of a future heart failure event or recurrence, reducing the severity of future heart failure, and/or preventing heart failure in an undiagnosed subjects. As such, the aromatic-cationic peptide D-Arg-2',6'-Dmt-Lys-Phe-NH₂ is useful for reducing cardiac troponin I levels, and preventing or treating heart failure in mammalian subjects.

### Example 11 - Effects of Aromatic-Cationic Peptides on cardiolipin in a Dog Model of Heart Failure

In this Example, the effect of aromatic-cationic peptide such as D-Arg-2',6'-Dmt-Lys-Phe-NH₂ on cardiolipin levels in dogs with coronary microembolization-induced heart failure was investigated.

### Methods

Heart failure was induced in dogs via multiple sequential intracoronary microembolizations as described in Sabbah, et al., Am J Physiol. (1991) 260: H1379-84, herein incorporated by reference in its entirety. Half the dogs were subsequently treated with D-Arg-2'6'-Dmt-Lys-Phe-NH₂; the other half were treated with drug vehicle and served as controls. Peptide treatment was started upon induction of heart failure (HF), defined as left ventricular ejection fraction of approximately 30%. A daily dose of D-Arg-2',6'-Dmt-Lys-Phe-NH₂ (0.5 mg/kg/day) was administered intravenously.

At the end of the treatment phase (12 weeks), dogs in both the vehicle and treatment groups were sacrificed and a sample of heart muscle from the left ventricle was removed, washed with saline, and immediately frozen and stored at -80°C.

For cardiolipin analysis, lipids were extracted from the heart tissue sample with a chloroform/methanol solution (Bligh Dyer extraction). Individual lipid extracts were reconstituted with chloroform: methanol (1:1), flushed with N₂, and then stored at -20°C before analysis via electrospray ionization mass spectroscopy using a triple-quadruple mass spectrometer equipped with an automated nanospray apparatus. Enhanced multidimensional mass spectrometry-based shotgun lipidomics for cardiolipin was performed as described by Han, et al. (2006) J Lipid Res 47(4): 864-879.

### Results

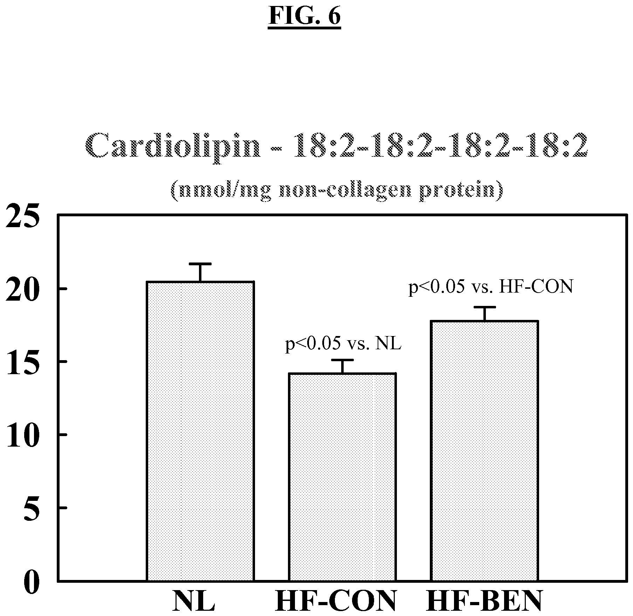

The 18:2 cardiolipin species was reduced in untreated HF dogs (HF-CON) (p < 0.05) as compared to normal cardiac tissue from normal dogs (NL). FIG. 1. However, HF dogs treated with D-Arg-2',6'-Dmt-Lys-Phe-NH₂ (HF-AP) had levels of 18:2 cardiolipin that were similar to NL dogs and greater that HF-CON (p < 0.05). **FIG. 6****.**

The 18:2 cardiolipin species is reduced in HF. The reduction of 18:2 cardiolipin leads to poor oxidative phosphorylation and subsequent LV dysfunction. Chronic treatment with D-Arg-2',6'-Dmt-Lys-Phe-NH₂ normalized 18:2 cardiolipin, which leads to improved LV function and rate of mitochondrial ATP synthesis.

These results show that aromatic-cationic peptides of the present invention, such as D-Arg-2',6'-Dmt-Lys-Phe-NH₂, or a pharmaceutically acceptable salt thereof, such as acetate or trifluoroacetate salt, are useful in the prevention and treatment of diseases and conditions associated with aberrant cardiolipin levels. In particular, these results show that aromatic-cationic peptides of the present invention, such as D-Arg-2',6'-Dmt-Lys-Phe-NH₂, or a pharmaceutically acceptable salt thereof, such as acetate or trifluoroacetate salt, are useful in increasing cardiolipin levels and for treating heart failure and related conditions.

### Example 12 - Effects of Aromatic-Cationic Peptides on MLCL AT1, ALCAT1, and Taz1 expression in a Dog Model of Heart Failure

In this Example, the effect of the aromatic-cationic peptide D-Arg-2',6'-Dmt-Lys-Phe-NH₂ on cardiolipin remodeling enzymes, MLCL AT1, ALCAT1, and Taz1 in dogs with coronary microembolization-induced heart failure was investigated.

### Methods

Heart failure was induced in dogs via multiple sequential intracoronary microembolizations as described in Sabbah, et al., Am J Physiol. (1991) 260:H1379-84, herein incorporated by reference in its entirety.

Twelve dogs were subject to coronary microembolization-induced heart failure (LV ejection fraction ∼30%) as described in Example 1. Subjects were randomized into D-Arg-2',6'-Dmt-Lys-Phe-NH₂-treated and control groups for a three-month trial. Subjects received 3 months of therapy with subcutaneous injections of D-Arg-2',6'-Dmt-Lys-Phe-NH₂ (0.5 mg/kg once daily, n=6) or saline (Untreated-HF Control, n=6). RNA was prepared from LV tissue of all 12 dogs at the end of the treatment phase and from the LV of 6 normal (NL) dogs for comparison. mRNA levels of Taz1, MLCL AT1 and ALCAT1 were determined by real-time PCR. Changes in mRNA expression were expressed as fold changes using the CT Method with normalization to glyceraldehyde 1,3 diphosphate dehydrogenase (GAPDH) as internal control.

### Results

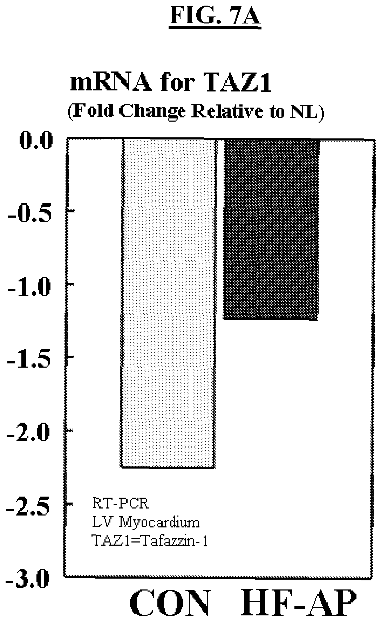

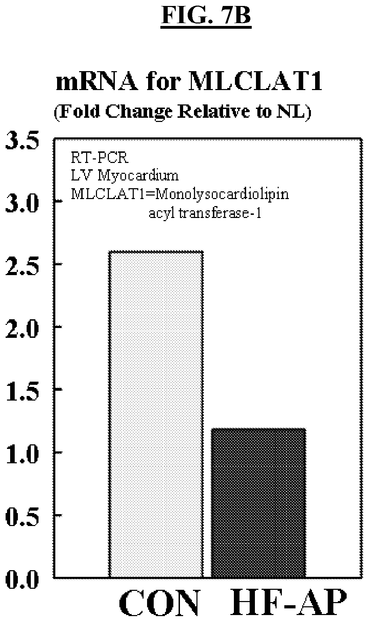

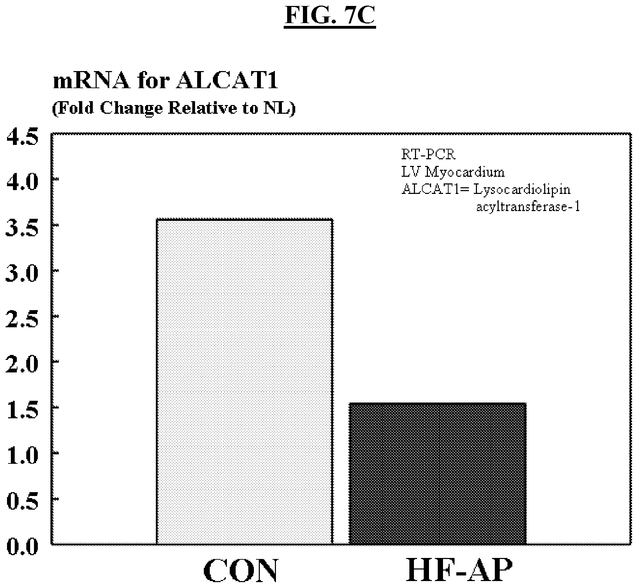

Compared to normal level (NL), mRNA levels of Taz1 in untreated HF dogs decreased 2.25-fold **(****FIG. 7A****)** while mRNA of MLCL AT1 and ALCAT1 increased 2.60-fold and 3.56-fold, respectively. **FIGs. 7B-C****.** Treatment with D-Arg-2'6'-Dmt-Lys-Phe-NH₂ attenuated the decrease of Taz1 1.23-fold and reduced the increase in MLCL AT1 and ALCAT1 1.18-fold and 1.54-fold, respectively. **FIGs. 7A-C****.**

HF is associated with deregulation of cardiolipin remodeling enzymes that can lead to pathologic remodeling of cardiolipin and to structural and functional mitochondrial abnormalities. Chronic therapy with D-Arg-2',6'-Dmt-Lys-Phe-NH₂ partially reverses these maladaptations thus allowing for resumption of physiologic post-biosynthesis remodeling of cardiolipin.

These results show that aromatic-cationic peptides of the present invention, such as D-Arg-2',6'-Dmt-Lys-Phe-NH₂, or a pharmaceutically acceptable salt thereof, such as acetate or trifluoroacetate salt, are useful in the prevention and treatment of diseases and conditions associated with aberrant cardiolipin remodeling enzyme levels, *e.g.,* MLCL AT1, ALCAT 1, and Taz1. In particular, these results show that aromatic-cationic peptides of the present invention, such as D-Arg-2',6'-Dmt-Lys-Phe-NH₂, or a pharmaceutically acceptable salt thereof, such as acetate or trifluoroacetate salt, are useful in methods for normalization of cardiolipin remodeling enzyme levels, *e.g.*, decreasing MLCL AT 1, ALCAT 1, and increasing Taz1 expression levels, and for treating heart failure and related conditions.

### EQUIVALENTS

The present invention is not to be limited in terms of the particular embodiments described in this application, which are intended as single illustrations of individual aspects of the invention. Many modifications and variations of this invention can be made without departing from its scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

As will be understood by one skilled in the art, for any and all purposes, particularly in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, *etc.* As a nonlimiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into sub-ranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

Other embodiments are set forth within the following claims.

## Claims

1. A composition for use in preventing or reducing the risk of heart failure in a human subject in need thereof, wherein the subject has higher than a control or "normal" level of ALCAT 1, wherein the composition comprises the peptide D-Arg-2',6'-Dmt-Lys-Phe-NH₂ or a pharmaceutically acceptable salt thereof.

2. The composition for use according to claim 1, wherein the heart failure results from hypertension; ischemic heart disease; exposure to a cardiotoxic compound; myocarditis; thyroid disease; viral infection; gingivitis; drug abuse; alcohol abuse; pericarditis; atherosclerosis; vascular disease; hypertrophic cardiomyopathy; acute myocardial infarction; left ventricular systolic dysfunction; coronary bypass surgery; starvation; or an eating disorder.

3. The composition for use according to any one of claims 1-2, wherein the peptide is intended to be administered orally, topically, systemically, intravenously, subcutaneously, intraperitoneally, or intramuscularly

4. The composition for use according to any one of claims 1-3, further comprising wherein the composition is intended to be separately, sequentially, or simultaneously administered with a cardiovascular agent to the subject.

5. The composition for use according to claim 4, wherein the cardiovascular agent is selected from the group consisting of: an anti-arrhythmia agent, a vasodilator, an anti-anginal agent, a corticosteroid, a cardioglycoside, a diuretic, a sedative, an angiotensin converting enzyme (ACE) inhibitor, an angiotensin II antagonist, a thrombolytic agent, a calcium channel blocker, a throboxane receptor antagonist, a radical scavenger, an antiplatelet drug, a β-adrenaline receptor blocking drug, α-receptor blocking drug, a sympathetic nerve inhibitor, a digitalis formulation, an inotrope, and an antihyperlipidemic drug.

6. The composition for use according to any one of claims 1-5, wherein the pharmaceutically acceptable salt comprises acetate or trifluoroacetate salt.

7. The composition for use according to claim 1, wherein the subject has suffered acute myocardial infarction.

8. The composition for use according to claim 1, wherein control or normal levels of ALCAT1 are defined by a population that is matched by age, ethnicity, weight and/or sex.

## Patentansprüche

1. Zusammensetzung zur Verwendung bei der Prävention oder Verringerung des Risikos von Herzinsuffizienz in einem menschlichen Individuum, bei dem diesbezüglicher Bedarf besteht, wobei das Individuum ein höheres Niveau von ALCAT1 als ein Kontrollniveau oder "normales" Niveau aufweist, wobei die Zusammensetzung das Peptid D-Arg-2',6'-Dmt-Lys-Phe-NH₂ oder ein pharmazeutisch verträgliches Salz davon umfasst.

2. Zusammensetzung zur Verwendung nach Anspruch 1, wobei die Herzinsuffizienz aus Bluthochdruck; ischämischer Herzerkrankung; Exposition gegenüber einer kardiotoxischen Verbindung; Myokarditis; Schilddrüsenerkrankung; Virusinfektion; Gingivitis; Drogen-/Arzneimittelmissbrauch; Alkoholmissbrauch; Perikarditis; Atherosklerose; Gefäßerkrankung; hypertropher Kardiomyopathie; akutem Myokardinfarkt; linksventrikulärer systolischer Dysfunktion; koronarer Bypass-Operation; Verhungern oder einer Essstörung resultiert.

3. Zusammensetzung zur Verwendung nach einem der Ansprüche 1-2, wobei das Peptid zur oralen, topischen, systemischen, intravenösen, subkutanen, intraperitonealen oder intramuskulären Verabreichung vorgesehen ist.

4. Zusammensetzung zur Verwendung nach einem der Ansprüche 1-3, ferner umfassend, dass die Zusammensetzung zur separaten, aufeinanderfolgenden oder gleichzeitigen Verabreichung mit einem Herz-Kreislauf-Mittel an das Individuum vorgesehen ist.

5. Zusammensetzung zur Verwendung nach Anspruch 4, wobei das Herz-Kreislauf-Mittel ausgewählt ist aus der Gruppe bestehend aus: einem Mittel gegen Arrhythmie, einem Vasodilatator, einem Mittel gegen Angina, einem Kortikosteroid, einem Kardioglykosid, einem Diuretikum, einem Sedativum, einem ACE-Inhibitor (ACE = Angtiotensin Converting Enzyme), einem Angiotensin-II-Antagonisten, einem Thrombolytikum, einem Calciumkanalblocker, einem Thromboxanrezeptor-Antagonisten, einem Radikalfänger, einem Arzneimittel gegen Thrombozyten, einem β-Adrenalinrezeptor-Blocker, einem α-Rezeptor-Blocker, einem Inhibitor sympathischer Nerven, einer Digitalisformulierung, einem Inotrop und einem antihyperlipidämischen Arzneimittel.

6. Zusammensetzung zur Verwendung nach einem der Ansprüche 1-5, wobei das pharmazeutisch verträgliche Salz Acetat- oder Trifluoracetat-Salz umfasst.

7. Zusammensetzung zur Verwendung nach Anspruch 1, wobei das Individuum einen akuten Myokardinfarkt erlitten hat.

8. Zusammensetzung zur Verwendung nach Anspruch 1, wobei Kontrollniveaus oder normale Niveaus von ALCAT1 durch eine nach Alter, Ethnizität, Gewicht und/oder Geschlecht angeglichene Population definiert sind.

## Revendications

1. Composition destinée à être utilisée en prévention ou réduction du risque d'insuffisance cardiaque chez un sujet humain qui en a besoin, le sujet ayant un taux plus élevé qu'un taux de référence ou « normal » d'ALCAT1, la composition comprenant le peptide D-Arg-2',6'-Dmt-Lys-Phe-NH₂ ou un sel pharmaceutiquement acceptable de celui-ci.

2. Composition destinée à être utilisée selon la revendication 1, l'insuffisance cardiaque résultant d'une hypertension ; d'une maladie cardiaque ischémique ; de l'exposition à un composé cardiotoxique ; d'une myocardite ; d'une maladie thyroïdienne ; d'une infection virale ; d'une gingivite ; d'une toxicomanie ; de l'abus d'alcool ; d'une péricardite ; d'une athérosclérose ; d'une maladie vasculaire ; d'une cardiomyopathie hypertrophique ; d'un infarctus du myocarde aigu ; d'un dysfonctionnement systolique du ventricule gauche ; d'une chirurgie de pontage coronarien ; d'une inanition ; ou d'un trouble de l'alimentation.

3. Composition destinée à être utilisée selon l'une quelconque des revendications 1-2, le peptide étant destiné à être administré par voie orale, topique, systémique, intraveineuse, sous-cutanée, intrapéritonéale ou intramusculaire.

4. Composition destinée à être utilisée selon l'une quelconque des revendications 1-3, la composition étant en outre destinée à être administrée séparément, séquentiellement ou simultanément avec un agent cardiovasculaire au sujet.

5. Composition destinée à être utilisée selon la revendication 4, l'agent cardiovasculaire étant choisi dans le groupe constitué par : un agent antiarythmique, un vasodilatateur, un agent antiangoreux, un corticostéroïde, un cardioglycoside, un diurétique, un sédatif, un inhibiteur de l'enzyme de conversion de l'angiotensine (ECA), un antagoniste de l'angiotensine II, un agent thrombolytique, un bloqueur des canaux calciques, un antagoniste des récepteurs de thromboxanes, un piégeur de radicaux libres, un médicament antiagrégant plaquettaire, un médicament bloquant les récepteurs β-adrénergiques, un médicament bloquant les récepteurs a, un inhibiteur des nerfs sympathiques, une formulation dérivée de digitale, un inotrope et un médicament antihyperlipidémiant.

6. Composition destinée à être utilisée selon l'une quelconque des revendications 1-5, le sel pharmaceutiquement acceptable comprenant un sel de type acétate ou trifluoroacétate.

7. Composition destinée à être utilisée selon la revendication 1, le sujet ayant souffert d'un infarctus du myocarde aigu.

8. Composition destinée à être utilisée selon la revendication 1, les taux de référence ou normaux d'ALCAT1 étant définis par une population qui correspond en âge, ethnicité, poids et/ou sexe.
